# EUROPEAN PATENT APPLICATION

(11) **EP 2 272 974 A1**
(43) Date of publication of application: **12.01.2011**
(21) Application number: 09724943.7
(22) Date of filing: 25.03.2009
(51) Int. Cl.: C12Q 1/68, C12N 15/09

(54) **METHOD FOR DETERMINATION OF DNA METHYLATION**

(30) Priority: 25.03.2008 JP 2008077966
(71) Applicant: Sumitomo Chemical Company, Limited, Tokyo 104-8260 (JP)
(72) Inventor: TOMIGAHARA, Yoshitaka, Toyonaka-shi Osaka 560-0013 (JP); SATOH, Hideo, Ibaraki-shi Osaka 567-0826 (JP); TARUI, Hirokazu, Toyonaka-shi Osaka 561-0802 (JP)
(74) Representative: Duckworth, Timothy John
(86) International application number: PCT/JP2009/056782
(87) International publication number: WO 2009/119890

(57) **Abstract**

The present invention relates to a method of measuring the content of methylated DNA in a DNA region of interest in a genomic DNA contained in a biological specimen, and so on.

## Description

### TECHNICAL FIELD

The present invention relates to a method of measuring the content of methylated DNA in a DNA region of interest in a genomic DNA contained in a biological specimen, and so on.

### BACKGROUND ART

As a method for evaluating the methylation state of DNA in an objective DNA region in a genomic DNA contained in a biological specimen, for example, there is known a method of measuring the content of methylated DNA in an objective DNA region in a genomic DNA (see, for example, Nucleic Acids Res., 1994, Aug 11; 22(15): 2990-7, and Proc. Natl. Acad. Sci. U.S.A., 1997, Mar 18; 94(6): 2284-9 for reference). In such a measuring method, first, it is necessary to extract DNA containing the objective DNA region from a DNA sample derived from a genomic DNA, and the extracting operation is complicated.

As a method of measuring the content of methylated DNA in an objective region of extracted DNA, for example, (1) a method of amplifying an objective region by subjecting the DNA to a chain reaction for DNA synthesis by DNA polymerase after modification of the DNA with a sulfite or the like (Polymerase Chain Reaction; hereinafter also referred to as PCR), and (2) a method of amplifying an objective region by subjecting the DNA to PCR after digestion of the DNA using a methylation sensitive restriction enzyme are known. Both of these methods require time and labor for DNA modification for detection of methylation, subsequent purification of the product, preparation of a reaction system for PCR, and checking of DNA amplification.

### DISCLOSURE OF THE INVENTION

It is an object of the present invention to provide a method of measuring the content of methylated DNA in an objective DNA region in a genomic DNA contained in a biological specimen in a simple and convenient manner.

That is, the present invention provides the following inventions.

### [Invention 1]

A method of measuring the content of methylated DNA in an objective DNA region in genomic DNA contained in a biological specimen, comprising:
(1) First step of subjecting a DNA sample derived from genomic DNA contained in a biological specimen to a digestion treatment with a methylation-sensitive restriction enzyme;
(2) Second step of obtaining a single-stranded DNA (plus strand) containing the objective DNA region from the DNA sample that has been subjected to the digestion treatment and obtained in First step, and causing base-pairing between the single-stranded DNA (plus strand) and a single-stranded immobilized oligonucleotide comprising a nucleotide sequence complementary to a part (provided that, not containing the objective DNA region) of 3'-end of the single-stranded DNA, thereby selecting the single-stranded DNA,
(3) Third step of extensionally-forming a double-stranded DNA from the single-stranded DNA by allowing a single extension of a primer using the single-stranded DNA selected in Second step as a template and the single-stranded immobilized oligonucleotide as the primer, and
(4) Fourth step comprising, as a pre step of the following regular steps, a step (pre step) of temporarily separating the extensionally-formed double-stranded DNA obtained in Third step into a single-stranded state, and as regular steps:
   (a) Step A (regular step) comprising Step A1 of selecting the DNA in a single-stranded state by causing base-pairing between the generated DNA in a single-stranded state (plus strand) and the single-stranded immobilized oligonucleotide (minus strand), and Step A2 of extensionally-forming double-stranded DNA from the DNA in a single-stranded state by causing one extension of a primer by using the DNA in a single-stranded state selected in Step A1 as a template and the single-stranded immobilized oligonucleotide as the primer, and
   (b) Step B (regular step) of extensionally-forming double-stranded DNA from the DNA in a single-stranded state by causing one extension of an extension primer by using the generated DNA in a single-stranded state (minus strand) as a template, and a primer (reverse primer) comprising a nucleotide sequence (plus strand) complementary to a partial nucleotide sequence (minus strand) of the nucleotide sequence of the DNA in a single-stranded state (minus strand), wherein the partial nucleotide sequence (minus strand) is located on further 3'-end side than the 3'-end of the nucleotide sequence (minus strand) complementary to the nucleotide sequence (plus strand) of the objective DNA region, as the extension primer,
   wherein the methylated DNA in the objective DNA region is amplified to a detectable level by repeating each regular step after temporarily separating the extensionally-formed double-stranded DNA obtained in the regular steps into a single-stranded state, and an amount of the amplified DNA is quantified.

### [Invention 2]

The method according to Invention 1, wherein in Second step, base-pairing is conducted in a reaction system containing a divalent cation when the single-stranded DNA containing the objective DNA region (plus strand) and a single-stranded immobilized oligonucleotide comprising a nucleotide sequence complementary to a part (provided that, not containing the objective DNA region) of the 3'-end of the single-stranded DNA are base-paired.

### [Invention 3]

The method according to Invention 2, wherein the divalent cation is a magnesium ion.

### [Invention 4]

The method further comprising prior to the pre step in Fourth step:
a step (Additional pre step) of adding into the reaction system a single-stranded oligonucleotide (minus strand) in a free state comprising a nucleotide sequence complementary to a part (provided that, not containing the objective DNA region) of 3'-end of the single-stranded DNA (plus strand) containing the objective DNA region, and further comprising the following one step as each regular step in Fourth step:
   (c) Step C (regular step) comprising:
      (i) Step C1 of selecting the DNA in a single-stranded state by base-pairing between the generated DNA in a single-stranded state (plus strand) and the single-stranded oligonucleotide (minus strand) added into the reaction system in Additional pre step, and
      (ii) Step C2 of extensionally-forming double-stranded DNA from the DNA in a single-stranded state by allowing one extension of a primer by using the DNA in a single-stranded state selected in Step C1 as a template and the single-stranded oligonucleotide (minus strand) as the primer.

### [Invention 5]

The method further comprising after the pre step in Fourth step:
a step (Additional pre step) of adding into the reaction system a single-stranded oligonucleotide (minus strand) in a free state comprising a nucleotide sequence complementary to a part (provided that, not containing the objective DNA region) of 3'-end of the single-stranded DNA (plus strand) containing the objective DNA region, and
a step (Additional re-pre step) of temporarily separating the extensionally-formed double-stranded DNA which is an undigested substance obtained through Third step and the Additional pre step (extensionally-formed double-stranded DNA not containing an unmethylated CpG pair in the recognition site of the methylation sensitive restriction enzyme) into a single-stranded state, and further comprising the following one step as each regular step in Fourth step:
   (c) Step C (Regular step) comprising:
      (i) Step C1 of selecting the DNA in a single-stranded state by base-pairing between the generated DNA in a single-stranded state (plus strand) and the single-stranded oligonucleotide (minus strand) added into the reaction system in Additional pre step, and
      (ii) Step C2 of extensionally-forming double-stranded DNA from the DNA in a single-stranded state by allowing one extension of a primer by using the DNA in a single-stranded state selected in Step C1 as a template and the single-stranded oligonucleotide (minus strand) as the primer.

### [Invention 6]

A method of measuring a methylation rate further comprising the following two steps as steps of the method according to any one of Inventions 1 to 5:
(5) Fifth step of amplifying DNA (total amount of methylated DNA and unmethylated DNA) of the objective DNA region to a detectable level by conducting Second to Fourth steps in the method of any one of Inventions 1 to 5 without conducting First step in the method of any one of Inventions 1 to 5, and quantifying the amplified DNA, and
(6) Sixth step of calculating a rate of methylated DNA in the objective DNA region based on a difference obtained by comparing the DNA amount quantified by Fourth step of any one of Inventions 1 to 5, and the DNA amount quantified in Fifth step.

### [Invention 7]

The method according to any one of Inventions 1 to 6, wherein the biological specimen is mammalian serum or plasma.

### [Invention 8]

The method according to any one of Inventions 1 to 6, wherein the biological specimen is mammalian blood or bodily fluid.

### [Invention 9]

The method according to any one of Inventions 1 to 6, wherein the biological specimen is a cell lysate or a tissue lysate.

### [Invention 10]

The method according to any one of Inventions 1 to 9, wherein the DNA sample derived from the genomic DNA contained in the biological specimen is a DNA sample digested in advance with a restriction enzyme the recognition cleavage site for which is not present in the objective DNA region possessed by the genomic DNA.

### [Invention 11]

The method according to any one of Inventions 1 to 10, wherein the DNA sample derived from genomic DNA contained in a biological specimen is a DNA sample purified in advance.

### [Invention 12]

The method according to any one of Inventions 1 to 11, wherein the digestion treatment with a methylation sensitive restriction enzyme is a digestion treatment comprising First (A) step of mixing a single-stranded DNA (plus strand) containing an objective DNA region with a masking oligonucleotide comprising a nucleotide sequence complementary to a nucleotide sequence of a recognition site of the methylation sensitive restriction enzyme, thereby generating single-stranded DNA in which the recognition site of the methylation sensitive restriction enzyme is protected, and First (B) step of digesting the single-stranded DNA selected in First (A) step with the methylation sensitive restriction enzyme.

### [Invention 13]

The method according to any one of Inventions 1 to 12, wherein the methylation sensitive restriction enzyme is a restriction enzyme the recognition cleavage site for which is present in the objective DNA region possessed by genomic DNA contained in a biological specimen.

### [Invention 14]

The method according to any one of Inventions 1 to 13, wherein the methylation sensitive restriction enzyme is HpaII or HhaI.

### [Invention 15]

A method of measuring the content of methylated DNA in an objective DNA region possessed by genomic DNA contained in a biological specimen, comprising:
(1) First step of subjecting a DNA sample derived from genomic DNA contained in a biological specimen to a digestion treatment with a methylation sensitive restriction enzyme;
(2) Second step of obtaining single-stranded DNA (plus strand) containing the objective DNA region from the DNA sample that has been subjected to the digestion treatment and obtained in First step, and causing base-pairing between the single-stranded DNA (plus strand) and a single-stranded immobilized oligonucleotide comprising a nucleotide sequence complementary to a part of the single-stranded DNA, thereby selecting the single-stranded DNA,
(3) Third step comprising a step of temporarily separating the single-stranded DNA selected in Second step into a single-stranded state, and extensionally-forming double-stranded DNA from the single-stranded DNA by allowing one extension of a primer using the generated DNA in a single-stranded state (plus strand) as a template and a forward primer comprising a nucleotide sequence (minus strand) complementary to a partial nucleotide sequence (plus strand) located on further 3'-end side than the 3'-end of the nucleotide sequence (plus strand) of the objective DNA region as an extension primer,
(4) Fourth step comprising, as a pre step of each of the following regular steps, a step (pre step) of temporarily separating the extensionally-formed double-stranded DNA obtained in Third step into a single-stranded state, and as regular steps:
   (a) Step A (Regular step) comprising Step A1 of selecting the DNA in a single-stranded state by causing base-pairing between the generated DNA in a single-stranded state (plus strand) and the forward primer (minus strand), and Step A2 of extensionally-forming double-stranded DNA from the DNA in a single-stranded state by causing one extension of a primer by using the DNA in a single-stranded state selected in Step A1 as a template and the forward primer as an extension primer, and
   (b) Step B (Regular step) of extensionally-forming double-stranded DNA from the DNA in a single-stranded state by causing one extension of an extension primer by using the generated DNA in a single-stranded state (minus strand) as a template, and a reverse primer comprising a nucleotide sequence (plus strand) complementary to a partial nucleotide sequence (minus strand) of the nucleotide sequence of the DNA in a single-stranded state (minus strand), wherein the partial nucleotide sequence (minus strand) is located on further 3'-end side than the 3'-end of the nucleotide sequence (minus strand) complementary to the nucleotide sequence (plus strand) of the objective DNA region, as the extension primer,
   wherein the methylated DNA in the objective DNA region is amplified to a detectable level by repeating each regular step in Fourth step after temporarily separating the extensionally-formed double-stranded DNA obtained in the regular steps into a single-stranded state, and an amount of the amplified DNA is quantified.

### [Invention 16]

The method according to Invention 15, wherein the single-stranded immobilized oligonucleotide is a single-stranded immobilized oligonucleotide immobilized at 5'- or 3'-end.

### [Invention 17]

The method according to Invention 15 or 16, wherein in Second step, base-pairing is conducted in a reaction system containing a divalent cation when the single-stranded DNA containing the objective DNA region (plus strand) and a single-stranded immobilized oligonucleotide comprising a nucleotide sequence complementary to a part (provided that, not containing the objective DNA region) of the 3'-end of the single-stranded DNA are base-paired.

### [Invention 18]

A method of measuring a methylation rate further comprising the following two steps as steps of the method of Invention 15 or 16:
(5) Fifth step of amplifying DNA (total amount of methylated DNA and unmethylated DNA) of the objective DNA region to a detectable level by conducting Second to Fourth steps in the method of any one of Inventions 1 to 5 without conducting First step in the method of Invention 15 or 16, and quantifying the amplified DNA, and
(6) Sixth step of calculating a rate of methylated DNA in the objective DNA region based on a difference obtained by comparing the DNA amount quantified in Fourth step of Invention 15 or 16, and the DNA amount quantified in Fifth step.

### [Invention 19]

The method according to Invention 15 or 16, wherein the biological specimen is mammalian serum or plasma.

### [Invention 20]

The method according to Invention 15 or 16, wherein the biological specimen is mammalian blood or bodily fluid.

### [Invention 21]

The method according to Invention 15 or 16, wherein the biological specimen is a cell lysate or a tissue lysate.

### [Invention 22]

The method according to Invention 15 or 16, wherein the DNA sample derived from the genomic DNA contained in the biological specimen is a DNA sample digested in advance with a restriction enzyme the recognition cleavage site for which is not present in the objective DNA region possessed by the genomic DNA.

### [Invention 23]

The method according to Invention 15 or 16, wherein the DNA sample derived from genomic DNA contained in a biological specimen is a DNA sample purified in advance.

### [Invention 24]

The method according to Invention 15 or 16, wherein the digestion treatment with a methylation sensitive restriction enzyme is a digestion treatment comprising First (A) step of mixing a single-stranded DNA (plus strand) containing an objective DNA region with a masking oligonucleotide comprising a nucleotide sequence complementary to a nucleotide sequence of a recognition site of the methylation sensitive restriction enzyme, thereby generating single-stranded DNA in which the recognition site of the methylation sensitive restriction enzyme is protected, and First (B) step of digesting the single-stranded DNA selected in First (A) step with the methylation sensitive restriction enzyme.

### [Invention 25]

The method according to Invention 15 or 16, wherein the methylation sensitive restriction enzyme is a restriction enzyme the recognition cleavage site for which is present in the objective DNA region possessed by genomic DNA contained in a biological specimen.

### [Invention 26]

The method according to Invention 15 or 16, wherein the methylation sensitive restriction enzyme is HpaII or HhaI.

### BRIEF DESCRIPTION OF THE DRAWINGS

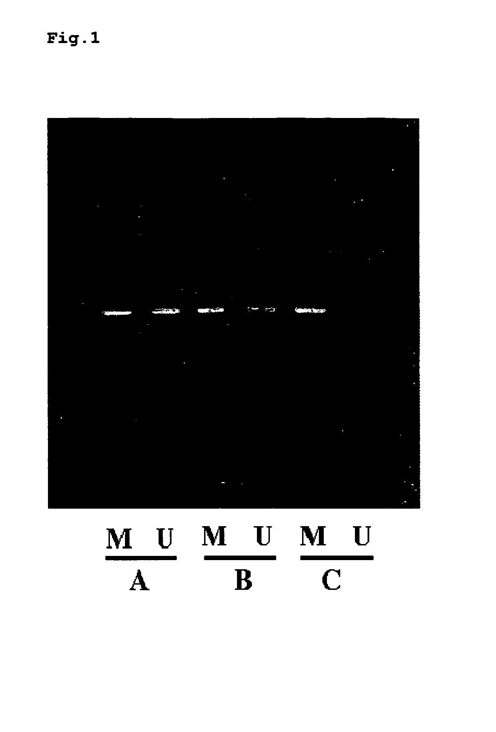

Fig. 1 shows results of 2% agarose gel electrophoresis of amplified products which are obtained by conducting a treatment of either "A (no treatment)", "B (HpaII treatment)" or "C (addition of masking oligonucleotide + HpaII treatment)" on the sample prepared in Example, and amplifying DNA in the region having the nucleotide sequence of SEQ ID NO:7 by PCR.

In the drawing, results of a sample subjected to "A" treatment of methylated oligonucleotide GPR7-2079-2176/98mer-M(7) (M) in which the HpaII recognition sequence is methylated, a sample subjected to "A" treatment of unmethylated oligonucleotide GPR7-2079-2176/98mer-UM (U) in which the HpaII recognition sequence is not methylated, a sample subjected to "B" treatment of methylated oligonucleotide GPR7-2079-2176/98mer-M(7) (M) in which the HpaII recognition sequence is methylated, a sample subjected to "B" treatment of unmethylated oligonucleotide GPR7-2079-2176/98mer-UM (U) in which the HpaII recognition sequence is not methylated, a sample subjected to "C" treatment of methylated oligonucleotide GPR7-2079-2176/98mer-M(7) (M) in which the HpaII recognition sequence is methylated, and a sample subjected to "C" treatment of unmethylated oligonucleotide GPR7-2079-2176/98mer-UM (U) in which the HpaII recognition sequence is not methylated are shown from the leftmost lane.

### MODE FOR CARRYING OUT THE INVENTION

As the "biological specimen" in the present invention, for example, a cell lysate, a tissue lysate (here the term "tissue" is used in a broad sense including blood, lymph node and so on) or biological samples including bodily fluids such as plasma, serum and lymph, and bodily secretions (urine, milk and so on) and genomic DNA extracted from these biological samples in mammals can be recited. The biological specimen may contain microorganism or virus. Therefore, by the term "genomic DNA" in the present invention, not only genomic DNA of biological specimen itself, but also genomic DNA of microorganism or virus contained in the biological specimen is implied. When the specimen derived from a mammal is blood, use of the present invention in a regular health check or a simple examination is expected.

For obtaining a genomic DNA from a specimen derived from a mammal, for example, DNA may be extracted using a commercially available DNA extraction kit. When blood is used as a specimen, plasma or serum is prepared from blood in accordance with a commonly used method, and using the prepared plasma or serum as a specimen, free DNA (including DNA derived from cancer cells such as gastric cancer cells) contained in the specimen is analyzed. This enables analysis of DNA derived from cancer cells such as gastric cancer cells while avoiding DNA derived from hemocytes, and improves the sensitivity of detection of cancer cells such as gastric cancer cells and a tissue containing the same.

The DNA sample derived from genomic DNA may be a DNA sample that is purified in advance by a prescribed method.

Usually, a gene (a genomic DNA) consists of four kinds of bases. In these bases, such a phenomenon is known that only cytosine is methylated, and such methylation modification of DNA is limited to cytosine in the nucleotide sequence represented by 5'-CG-3' (C represents cytosine, and G represents guanine. Hereinafter, the nucleotide sequence is also referred to as "CpG"). The site to be methylated in cytosine is its position 5. In DNA replication prior to cell division, only cytosine in "CpG" of a template chain is methylated immediately after replication, however, cytosine in "CpG" of a newly-generated strand is immediately methylated by the action of methyltransferase. Therefore, the methylation state of DNA will be passed to new two sets of DNA even after DNA replication. The term "methylated DNA" in the present invention means DNA occurring by such methylation modification.

The term "CpG pair" in the present invention means double-stranded oligonucleotide in which the nucleotide sequence represented by CpG and a CpG that is complement with this are base-paired.

The term "objective DNA region" (hereinafter, also referred to as an "objective region") used in the present invention means a DNA region for which presence or absence of methylation of cytosine included in the region is to be examined, and has a recognition site of one or more kinds of methylation sensitive restriction enzyme. A DNA region containing at least one cytosine in the nucleotide sequence represented by CpG which is present in a nucleotide sequence of a promoter region, an untranslated region, or a translated region (coding region) of a useful protein gene such as Lysyl oxidase, HRAS-like suppressor, bA305P22.2.1, Gamma filamin, HAND1, Homologue of RIKEN 2210016F16, FLJ32130, PPARG angiopoietin-related protein, Thrombomodulin, p53-responsive gene 2, Fibrillin2, Neurofilament3, disintegrin and metalloproteinase domain 23, G protein-coupled receptor 7, G-protein coupled somatostatin and angiotensin-like peptide receptor, Solute carrier family 6 neurotransmitter transporter noradrenalin member 2 and so on can be recited.

To be more specific, when the useful protein gene is a Lysyl oxidase gene, as a nucleotide sequence that contains at least one nucleotide sequence represented by CpG present in a nucleotide sequence of its promoter region, untranslated region or translated region (coding region), a nucleotide sequence of a genomic DNA containing exon 1 of a Lysyl oxidase gene derived from human, and a promoter region located 5' upstream of the same can be recited, and more concretely, the nucleotide sequence of SEQ ID NO: 1 (corresponding to the nucleotide sequence represented by base No. 16001 to 18661 in the nucleotide sequence described in Genbank Accession No. AF270645) can be recited. In the nucleotide sequence of SEQ ID NO: 1, ATG codon encoding methionine at amino terminal of Lysyl oxidase protein derived from human is represented in base No. 2031 to 2033, and a nucleotide sequence of the above exon 1 is represented in base No. 1957 to 2661. Cytosine in the nucleotide sequence represented by CpG which is present in the nucleotide sequence of SEQ ID NO: 1, in particular, cytosine in CpG which is present in a region where CpGs are densely present in the nucleotide sequence of SEQ ID NO: 1 exhibits high methylation frequency (namely, a high methylation state (hypermethylation)) in, for example, cancer cells such as gastric cancer cells. More concretely, as cytosine exhibiting high methylation frequency in gastric cancer cells, for example, cytosines represented by base Nos. 1539, 1560, 1574, 1600, 1623, 1635, 1644, 1654, 1661, 1682, 1686, 1696, 1717, 1767, 1774, 1783, 1785, 1787, 1795 and so on in the nucleotide sequence of SEQ ID NO: 1 can be recited.

To be more specific, when the useful protein gene is a HRAS-like suppressor gene, as a nucleotide sequence that contains at least one nucleotide sequence represented by CpG present in a nucleotide sequence of its promoter region, untranslated region or translated region (coding region), a nucleotide sequence of a genomic DNA containing exon 1 of a HRAS-like suppressor gene derived from human, and a promoter region located 5' upstream of the same can be recited, and more concretely, the nucleotide sequence of SEQ ID NO: 2 (corresponding to the nucleotide sequence represented by base No. 172001 to 173953 in the nucleotide sequence described in Genbank Accession No. AC068162) can be recited. In the nucleotide sequence of SEQ ID NO: 2, the nucleotide sequence of exon 1 of a HRAS-like suppressor gene derived from human is represented in base No. 1743 to 1953. Cytosine in the nucleotide sequence represented by CpG which is present in the nucleotide sequence of SEQ ID NO: 2, in particular, cytosine in CpG which is present in a region where CpGs are densely present in the nucleotide sequence of SEQ ID NO: 2 exhibits high methylation frequency (namely, a high methylation state (hypermethylation)) in, for example, cancer cells such as gastric cancer cells. More concretely, as cytosine exhibiting high methylation frequency in gastric cancer cells, for example, cytosines represented by base Nos. 1316, 1341, 1357, 1359, 1362, 1374, 1390, 1399, 1405, 1409, 1414, 1416, 1422, 1428, 1434, 1449, 1451, 1454, 1463, 1469, 1477, 1479, 1483, 1488, 1492, 1494, 1496, 1498, 1504, 1510, 1513, 1518, 1520 and so on in the nucleotide sequence of SEQ ID NO: 2 can be recited.

To be more specific, when the useful protein gene is a bA305P22.2.1 gene, as a nucleotide sequence that contains at least one nucleotide sequence represented by CpG present in a nucleotide sequence of its promoter region, untranslated region or translated region (coding region), a nucleotide sequence of a genomic DNA containing exon 1 of a bA305P22.2.1 gene derived from human, and a promoter region located 5' upstream of the same can be recited, and more concretely, the nucleotide sequence of SEQ ID NO: 3 (corresponding to the nucleotide sequence represented by base No. 13001 to 13889 in the nucleotide sequence described in Genbank Accession No. AL121673) can be recited. In the nucleotide sequence of SEQ ID NO: 3, ATG codon encoding methionine at amino terminal of bA305P22.2.1 protein derived from human is represented in base No. 849 to 851, and a nucleotide sequence of the above exon 1 is represented in base No. 663 to 889. Cytosine in the nucleotide sequence represented by CpG which is present in the nucleotide sequence of SEQ ID NO: 3, in particular, cytosine in CpG which is present in a region where CpGs are densely present in the nucleotide sequence of SEQ ID NO: 3 exhibits high methylation frequency (namely, a high methylation state (hypermethylation)) in, for example, cancer cells such as gastric cancer cells. More concretely, as cytosine exhibiting high methylation frequency in gastric cancer cells, for example, cytosines represented by base Nos. 329, 335, 337, 351, 363, 373, 405, 424, 427, 446, 465, 472, 486 and so on in the nucleotide sequence of SEQ ID NO: 3 can be recited.

To be more specific, when the useful protein gene is a Gamma filamin gene, as a nucleotide sequence that contains at least one nucleotide sequence represented by CpG present in a nucleotide sequence of its promoter region, untranslated region or translated region (coding region), a nucleotide sequence of a genomic DNA containing exon 1 of a Gamma filamin gene derived from human, and a promoter region located 5' upstream of the same can be recited, and more concretely, the nucleotide sequence of SEQ ID NO: 4 (corresponding to a complementary sequence to the nucleotide sequence represented by base No. 63528 to 64390 in the nucleotide sequence described in Genbank Accession No. AC074373) can be recited. In the nucleotide sequence of SEQ ID NO: 4, ATG codon encoding methionine at amino terminal of Gamma filamin protein derived from human is represented in base No. 572 to 574, and a nucleotide sequence of the above exon 1 is represented in base No. 463 to 863. Cytosine in the nucleotide sequence represented by CpG which is present in the nucleotide sequence of SEQ ID NO: 4, in particular, cytosine in CpG which is present in a region where CpGs are densely present in the nucleotide sequence of SEQ ID NO: 4 exhibits high methylation frequency (namely, a high methylation state (hypermethylation)) in, for example, cancer cells such as gastric cancer cells. More concretely, as cytosine exhibiting high methylation frequency in gastric cancer cells, for example, cytosines represented by base Nos. 329, 333, 337, 350, 353, 360, 363, 370, 379, 382, 384, 409, 414, 419, 426, 432, 434, 445, 449, 459, 472, 474, 486, 490, 503, 505 and so on in the nucleotide sequence of SEQ ID NO: 4 can be recited.

To be more specific, when the useful protein gene is a HAND1 gene, as a nucleotide sequence that contains at least one nucleotide sequence represented by CpG present in a nucleotide sequence of its promoter region, untranslated region or translated region (coding region), a nucleotide sequence of a genomic DNA containing exon 1 of a HAND1 gene derived from human, and a promoter region located 5' upstream of the same can be recited, and more concretely, the nucleotide sequence of SEQ ID NO: 5 (corresponding to a complementary sequence to the nucleotide sequence represented by base No. 24303 to 26500 in the nucleotide sequence described in Genbank Accession No. AC026688) can be recited. In the nucleotide sequence of SEQ ID NO: 5, ATG codon encoding methionine at amino terminal of HAND1 protein derived from human is represented in base No. 1656 to 1658, and a nucleotide sequence of the above exon 1 is represented in base No. 1400 to 2198. Cytosine in the nucleotide sequence represented by CpG which is present in the nucleotide sequence of SEQ ID NO: 5, in particular, cytosine in CpG which is present in a region where CpGs are densely present in the nucleotide sequence of SEQ ID NO: 5 exhibits high methylation frequency (namely, a high methylation state (hypermethylation)) in, for example, cancer cells such as gastric cancer cells. More concretely, as cytosine exhibiting high methylation frequency in gastric cancer cells, for example, cytosines represented by base Nos. 1153, 1160, 1178, 1187, 1193, 1218, 1232, 1266, 1272, 1292, 1305, 1307, 1316, 1356, 1377, 1399, 1401, 1422, 1434 and so on in the nucleotide sequence of SEQ ID NO: 5 can be recited.

To be more specific, when the useful protein gene is a Homologue of RIKEN 2210016F16 gene, as a nucleotide sequence that contains at least one nucleotide sequence represented by CpG present in a nucleotide sequence of its promoter region, untranslated region or translated region (coding region), a nucleotide sequence of a genomic DNA containing exon 1 of a Homologue of RIKEN 2210016F16 gene derived from human, and a promoter region located 5' upstream of the same can be recited, and more concretely, the nucleotide sequence of SEQ ID NO: 6 (corresponding to a complementary nucleotide sequence to the nucleotide sequence represented by base No. 157056 to 159000 in the nucleotide sequence described in Genbank Accession No. AL354733) can be recited. In the nucleotide sequence of SEQ ID NO: 6, a nucleotide sequence of exon 1 of a Homologue of a RIKEN 2210016F16 gene derived from human is represented in base No. 1392 to 1945. Cytosine in the nucleotide sequence represented by CpG which is present in the nucleotide sequence of SEQ ID NO: 6, in particular, cytosine in CpG which is present in a region where CpGs are densely present in the nucleotide sequence of SEQ ID NO: 6 exhibits high methylation frequency (namely, a high methylation state (hypermethylation)) in, for example, cancer cells such as gastric cancer cells. More concretely, as cytosine exhibiting high methylation frequency in gastric cancer cells, for example, cytosines represented by base Nos. 1172, 1175, 1180, 1183, 1189, 1204, 1209, 1267, 1271, 1278, 1281, 1313, 1319, 1332, 1334, 1338, 1346, 1352, 1358, 1366, 1378, 1392, 1402, 1433, 1436, 1438 and so on in the nucleotide sequence of SEQ ID NO: 6 can be recited.

To be more specific, when the useful protein gene is a FLJ32130 gene, as a nucleotide sequence that contains at least one nucleotide sequence represented by CpG present in a nucleotide sequence of its promoter region, untranslated region or translated region (coding region), a nucleotide sequence of a genomic DNA containing exon 1 of a FLJ32130 gene derived from human, and a promoter region located 5' upstream of the same can be recited, and more concretely, the nucleotide sequence of SEQ ID NO: 7 (corresponding to a complementary nucleotide sequence to the nucleotide sequence represented by base No. 1 to 2379 in the nucleotide sequence described in Genbank Accession No. AC002310) can be recited. In the nucleotide sequence of SEQ ID NO: 7, ATG codon encoding methionine at amino terminal of FLJ32130 protein derived from human is represented in base No. 2136 to 2138, and a nucleotide sequence assumed to be the above exon 1 is represented in base No. 2136 to 2379. Cytosine in the nucleotide sequence represented by CpG which is present in the nucleotide sequence of SEQ ID NO: 7, in particular, cytosine in CpG which is present in a region where CpGs are densely present in the nucleotide sequence of SEQ ID NO: 7 exhibits high methylation frequency (namely, a high methylation state (hypermethylation)) in, for example, cancer cells such as gastric cancer cells. More concretely, as cytosine exhibiting high methylation frequency in gastric cancer cells, for example, cytosines represented by base Nos. 1714, 1716, 1749, 1753, 1762, 1795, 1814, 1894, 1911, 1915, 1925, 1940, 1955, 1968 and so on in the nucleotide sequence of SEQ ID NO: 7 can be recited.

To be more specific, when the useful protein gene is a PPARG angiopoietin-related protein gene, as a nucleotide sequence that contains at least one nucleotide sequence represented by CpG present in a nucleotide sequence of its promoter region, untranslated region or translated region (coding region), a nucleotide sequence of a genomic DNA containing exon 1 of a PPARG angiopoietin-related protein gene derived from human, and a promoter region located 5' upstream of the same can be recited, and more concretely, the nucleotide sequence of SEQ ID NO: 8 can be recited. In the nucleotide sequence of SEQ ID NO: 8, ATG codon encoding methionine at amino terminal of PPARG angiopoietin-related protein derived from human is represented in base No. 717 to 719, and a nucleotide sequence of the 5'-end part of the above exon 1 is represented in base No. 1957 to 2661. Cytosine in the nucleotide sequence represented by CpG which is present in the nucleotide sequence of SEQ ID NO: 8, in particular, cytosine in CpG which is present in a region where CpGs are densely present in the nucleotide sequence of SEQ ID NO: 8 exhibits high methylation frequency (namely, a high methylation state (hypermethylation)) in, for example, cancer cells such as gastric cancer cells. More concretely, as cytosine exhibiting high methylation frequency in gastric cancer cells, for example, cytosines represented by base Nos. 35, 43, 51, 54, 75, 85, 107, 127, 129, 143, 184, 194, 223, 227, 236, 251, 258 and so on in the nucleotide sequence of SEQ ID NO: 8 can be recited.

To be more specific, when the useful protein gene is a Thrombomodulin gene, as a nucleotide sequence that contains at least one nucleotide sequence represented by CpG present in a nucleotide sequence of its promoter region, untranslated region or translated region (coding region), a nucleotide sequence of a genomic DNA containing exon 1 of a Thrombomodulin gene derived from human, and a promoter region located 5' upstream of the same can be recited, and more concretely, the nucleotide sequence of SEQ ID NO: 9 (corresponding to the nucleotide sequence represented by base No. 1 to 6096 in the nucleotide sequence described in Genbank Accession No. AF495471) can be recited. In the nucleotide sequence of SEQ ID NO: 9, ATG codon encoding methionine at amino terminal of Thrombomodulin protein derived from human is represented in base No. 2590 to 2592, and a nucleotide sequence of the above exon 1 is represented in base No. 2048 to 6096. Cytosine in the nucleotide sequence represented by CpG which is present in the nucleotide sequence of SEQ ID NO: 9, in particular, cytosine in CpG which is present in a region where CpGs are densely present in the nucleotide sequence of SEQ ID NO: 9 exhibits high methylation frequency (namely, a high methylation state (hypermethylation)) in, for example, cancer cells such as gastric cancer cells. More concretely, as cytosine exhibiting high methylation frequency in gastric cancer cells, for example, cytosines represented by base Nos. 1539, 1551, 1571, 1579, 1581, 1585, 1595, 1598, 1601, 1621, 1632, 1638, 1645, 1648, 1665, 1667, 1680, 1698, 1710, 1724, 1726, 1756 and so on in the nucleotide sequence of SEQ ID NO: 9 can be recited.

To be more specific, when the useful protein gene is a p53-responsive gene 2 gene, as a nucleotide sequence that contains at least one nucleotide sequence represented by CpG present in a nucleotide sequence of its promoter region, untranslated region or translated region (coding region), a nucleotide sequence of a genomic DNA containing exon 1 of a p53-responsive gene 2 gene derived from human, and a promoter region located 5' upstream of the same can be recited, and more concretely, the nucleotide sequence of SEQ ID NO: 10 (corresponding to a complementary sequence to the nucleotide sequence represented by base No. 113501 to 116000 in the nucleotide sequence described in Genbank Accession No. AC009471) can be recited. In the nucleotide sequence of SEQ ID NO: 10, a nucleotide sequence of exon 1 of a p53-responsive gene 2 gene derived from human is represented in base No. 1558 to 1808. Cytosine in the nucleotide sequence represented by CpG which is present in the nucleotide sequence of SEQ ID NO: 10 exhibits high methylation frequency (namely, a high methylation state (hypermethylation)) in, for example, cancer cells such as pancreas cancer cells. More concretely, as cytosine exhibiting high methylation frequency in pancreas cancer cells, for example, cytosines represented by base Nos. 1282, 1284, 1301, 1308, 1315, 1319, 1349, 1351, 1357, 1361, 1365, 1378, 1383 and so on in the nucleotide sequence of SEQ ID NO: 10 can be recited.

To be more specific, when the useful protein gene is a Fibrillin2 gene, as a nucleotide sequence that contains at least one nucleotide sequence represented by CpG present in a nucleotide sequence of its promoter region, untranslated region or translated region (coding region), a nucleotide sequence of a genomic DNA containing exon 1 of a Fibrillin2 gene derived from human, and a promoter region located 5' upstream of the same can be recited, and more concretely, the nucleotide sequence of SEQ ID NO: 11 (corresponding to a complementary sequence to the nucleotide sequence represented by base No. 118801 to 121000 in the nucleotide sequence described in Genbank Accession No. AC113387) can be recited. In the nucleotide sequence of SEQ ID NO: 11, a nucleotide sequence of exon 1 of a Fibrillin2 gene derived from human is represented in base No. 1091 to 1345. Cytosine in the nucleotide sequence represented by CpG which is present in the nucleotide sequence of SEQ ID NO: 11 exhibits high methylation frequency (namely, a high methylation state (hypermethylation)) in, for example, cancer cells such as pancreas cancer cells. More concretely, as cytosine exhibiting high methylation frequency in pancreas cancer cells, for example, cytosines represented by base Nos. 679, 687, 690, 699, 746, 773, 777, 783, 795, 799, 812, 823, 830, 834, 843 and so on in the nucleotide sequence of SEQ ID NO: 11 can be recited.

To be more specific, when the useful protein gene is a Neurofilament3 gene, as a nucleotide sequence that contains at least one nucleotide sequence represented by CpG present in a nucleotide sequence of its promoter region, untranslated region or translated region (coding region), a nucleotide sequence of a genomic DNA containing exon 1 of a Neurofilament3 gene derived from human, and a promoter region located 5' upstream of the same can be recited, and more concretely, the nucleotide sequence of SEQ ID NO: 12 (corresponding to a complementary sequence to the nucleotide sequence represented by base No. 28001 to 30000 in the nucleotide sequence described in Genbank Accession No. AF106564) can be recited. In the nucleotide sequence of SEQ ID NO: 12, a nucleotide sequence of exon 1 of a Neurofilament3 gene derived from human is represented in base No. 614 to 1694. Cytosine in the nucleotide sequence represented by CpG which is present in the nucleotide sequence of SEQ ID NO: 12 exhibits high methylation frequency (namely, a high methylation state (hypermethylation)) in, for example, cancer cells such as pancreas cancer cells. More concretely, as cytosine exhibiting high methylation frequency in pancreas cancer cells, for example, cytosines represented by base Nos. 428, 432, 443, 451, 471, 475, 482, 491, 499, 503, 506, 514, 519, 532, 541, 544, 546, 563, 566, 572, 580 and so on in the nucleotide sequence of SEQ ID NO: 12 can be recited.

To be more specific, when the useful protein gene is a disintegrin and metalloproteinase domain 23 gene, as a nucleotide sequence that contains at least one nucleotide sequence represented by CpG present in a nucleotide sequence of its promoter region, untranslated region or translated region (coding region), a nucleotide sequence of a genomic DNA containing exon 1 of a disintegrin and metalloproteinase domain 23 gene derived from human, and a promoter region located 5' upstream of the same can be recited, and more concretely, the nucleotide sequence of SEQ ID NO: 13 (corresponding to the nucleotide sequence represented by base No. 21001 to 23300 in the nucleotide sequence described in Genbank Accession No. AC009225) can be recited. In the nucleotide sequence of SEQ ID NO: 13, a nucleotide sequence of exon 1 of a disintegrin and metalloproteinase domain 23 gene derived from human is represented in base No. 1194 to 1630. Cytosine in the nucleotide sequence represented by CpG which is present in the nucleotide sequence of SEQ ID NO: 13 exhibits high methylation frequency (namely, a high methylation state (hypermethylation)) in, for example, cancer cells such as pancreas cancer cells. More concretely, as cytosine exhibiting high methylation frequency in pancreas cancer cells, for example, cytosines represented by base Nos. 998, 1003, 1007, 1011, 1016, 1018, 1020, 1026, 1028, 1031, 1035, 1041, 1043, 1045, 1051, 1053, 1056, 1060, 1066, 1068, 1070, 1073, 1093, 1096, 1106, 1112, 1120, 1124, 1126 and so on in the nucleotide sequence of SEQ ID NO: 13 can be recited.

To be more specific, when the useful protein gene is a G protein-coupled receptor 7 gene, as a nucleotide sequence that contains at least one nucleotide sequence represented by CpG present in a nucleotide sequence of its promoter region, untranslated region or translated region (coding region), a nucleotide sequence of a genomic DNA containing exon 1 of a G protein-coupled receptor 7 gene derived from human, and a promoter region located 5' upstream of the same can be recited, and more concretely, the nucleotide sequence of SEQ ID NO: 14 (corresponding to a nucleotide sequence represented by base No. 75001 to 78000 in the nucleotide sequence described in Genbank Accession No. AC009800) can be recited. In the nucleotide sequence of SEQ ID NO: 14, a nucleotide sequence of exon 1 of a G protein-coupled receptor 7 gene derived from human is represented in base No. 1666 to 2652. Cytosine in the nucleotide sequence represented by CpG which is present in the nucleotide sequence of SEQ ID NO: 14 exhibits high methylation frequency (namely, a high methylation state (hypermethylation)) in, for example, cancer cells such as pancreas cancer cells. More concretely, as cytosine exhibiting high methylation frequency in pancreas cancer cells, for example, cytosines represented by base Nos. 1480, 1482, 1485, 1496, 1513, 1526, 1542, 1560, 1564, 1568, 1570, 1580, 1590, 1603, 1613, 1620 and so on in the nucleotide sequence of SEQ ID NO: 14 can be recited.

To be more specific, when the useful protein gene is a G-protein coupled somatostatin and angiotensin-like peptide receptor gene, as a nucleotide sequence that contains at least one nucleotide sequence represented by CpG present in a nucleotide sequence of its promoter region, untranslated region or translated region (coding region), a nucleotide sequence of a genomic DNA containing exon 1 of a G-protein coupled somatostatin and angiotensin-like peptide receptor gene derived from human, and a promoter region located 5' upstream of the same can be recited, and more concretely, the nucleotide sequence of SEQ ID NO: 15 (corresponding to a complementary sequence to a nucleotide sequence represented by base No. 57001 to 60000 in the nucleotide sequence described in Genbank Accession No. AC008971) can be recited. In the nucleotide sequence of SEQ ID NO: 15, a nucleotide sequence of exon 1 of a G-protein coupled somatostatin and angiotensin-like peptide receptor gene derived from human is represented in base No. 776 to 2632. Cytosine in the nucleotide sequence represented by CpG which is present in the nucleotide sequence of SEQ ID NO: 15 exhibits high methylation frequency (namely, a high methylation state (hypermethylation)) in, for example, cancer cells such as pancreas cancer cells. More concretely, as cytosine exhibiting high methylation frequency in pancreas cancer cells, for example, cytosines represented by base Nos. 470, 472, 490, 497, 504, 506, 509, 514, 522, 540, 543, 552, 566, 582, 597, 610, 612 and so on in the nucleotide sequence of SEQ ID NO: 15 can be recited.

To be more specific, when the useful protein gene is a Sol ute carrier family 6 neurotransmitter transporter noradrenalin member 2 gene, as a nucleotide sequence that contains at least o ne nucleotide sequence represented by CpG present in a nucleotid e sequence of its promoter region, untranslated region or transl ated region (coding region), a nucleotide sequence of a genomic DNA containing exon 1 of a Solute carrier family 6 neurotransmit ter transporter noradrenalin member 2 gene derived from human, a nd a promoter region located 5' upstream of the same can be reci ted, and more concretely, the nucleotide sequence of SEQ ID NO: 16 (corresponding to a complementary sequence to a nucleotide se quence represented by base No. 78801 to 81000 in the nucleotide sequence described in Genbank Accession No. AC026802) can be rec ited. In the nucleotide sequence of SEQ ID NO: 16, a nucleotide sequence of exon 1 of a Solute carrier family 6 neurotransmitte r transporter noradrenalin member 2 gene derived from human is r epresented in base No. 1479 to 1804. Cytosine in the nucleotide sequence represented by CpG which is present in the nucleotide sequence of SEQ ID NO: 16 exhibits high methylation frequency (n amely, a high methylation state (hypermethylation)) in, for exa mple, cancer cells such as pancreas cancer cells. More concrete ly, as cytosine exhibiting high methylation frequency in pancre as cancer cells, for example, cytosines represented by base Nos. 1002, 1010, 1019, 1021, 1051, 1056, 1061, 1063, 1080, 1099, 111 0, 1139, 1141, 1164, 1169, 1184 and so on in the nucleotide sequ ence of SEQ ID NO: 16 can be recited.

In the present invention, the "amount of the amplified DNA (amplifying methylated DNA in the objective DNA region to a detectable level)" means an amount itself after amplification of methylated DNA in the objective region possessed by a genomic DNA contained in the biological specimen, namely, the amount determined in Fourth step of the present invention. For example, when the biological specimen is 1 mL of serum, it means the amount of DNA amplified based on the methylated DNA contained in 1 mL of serum.

The term "methylation rate" in the present invention means the numerical value obtained by dividing the amount after amplification of methylated DNA by a total of the amount of methylated DNA after amplification in the objective DNA region possessed by a genomic DNA contained in the biological specimen and the amount of unmethylated DNA after amplification.

In First step, a DNA sample derived from genomic DNA contained in a biological specimen is subjected to a digestion treatment with a methylation-sensitive restriction enzyme.

The "methylation-sensitive restriction enzyme" in the present invention, for example, a restriction enzyme or the like that does not digest a recognition sequence containing methylated cytosine, but digests only a recognition sequence containing unmethylated cytosine. In other words, in the case of DNA wherein cytosine contained in a recognition sequence inherently recognizable by the methylation sensitive restriction enzyme is methylated, the DNA will not be cleaved even when the methylation sensitive restriction enzyme is caused to act on the DNA. On the other hand, in the case of DNA wherein cytosine contained in a recognition sequence inherently recognizable by the methylation sensitive restriction enzyme is not methylated, the DNA will be cleaved when the methylation sensitive restriction enzyme is caused to act on the DNA. Concrete examples of such methylation sensitive restriction enzymes include HpaII, BstUI, NarI, SacII, and HhaI. The aforementioned methylation sensitive restriction enzymes have already been revealed by Gruenbaum et al. (Nucleic Acid Research, 9, 2509-2515).

As a method of examining whether or not digestion by the methylation sensitive restriction enzyme occurs, concretely, for example, a method of conducting PCR using a pair of primers capable of amplifying DNA containing cytosine which is a target of analysis in a recognition sequence while using the DNA as a template, and examining whether or not the DNA is amplified (amplified product) can be recited. When the cytosine which is a target of analysis is methylated, an amplified product is obtained. On the other hand, when the cytosine which is a target of analysis is not methylated, an amplified product is not obtained. In this manner, by comparing the amounts of amplified DNA, it is possible to measure the methylated rate of the cytosine which is a target of analysis. In brief, when genomic DNA contained in the biological specimen is methylated, it is possible to distinguish whether or not cytosine in CpG pair existing in the recognition site of the methylation sensitive restriction enzyme in genomic DNA contained in the biological specimen is methylated by utilizing the characteristic that the methylation sensitive restriction enzyme fails to cleave methylated DNA. In other words, when cytosine in at least one CpG pair existing in the recognition site of the methylation sensitive restriction enzyme in genomic DNA contained in the biological specimen is not methylated, the DNA having such a recognition site will be cleaved by the methylation sensitive restriction enzyme when it is subjected to a digestion treatment with the methylation sensitive restriction enzyme. Further, when cytosine in every CpG pair existing in the recognition site of the methylation sensitive restriction enzyme in genomic DNA contained in the biological specimen is methylated, the DNA having such a recognition site will not be cleaved by the methylation sensitive restriction enzyme. Therefore, by conducting PCR using a pair of primers capable of amplifying the objective DNA region as will be described later after executing the digestion treatment, an amplified product by PCR will not be obtained when cytosine in at least one CpG pair existing in the recognition site of the methylation sensitive restriction enzyme in genomic DNA contained in the biological specimen is not methylated, and on the other hand, an amplified product by PCR will be obtained when cytosine in every CpG pair existing in the recognition site of the methylation sensitive restriction enzyme in genomic DNA contained in the biological specimen is methylated.

Concretely, First step may be executed, for example, in the following manner when genomic DNA contained in the biological specimen is genomic DNA from mammals. Genomic DNA from mammals is added with 3 µL of an optimum 10 × buffer (330 mM Tris-Acetate pH 7.9, 660 mM KOAc, 100 mM MgOAc₂, 5 mM Dithiothreitol), 3 µL of 1 mg/mL BSA aqueous solution, each 1. 5 µL of a methylation sensitive restriction enzyme such as HpaII or HhaI (10 U/µL), and the resultant mixture is added with sterilized ultrapure water to make the liquid amount 30 µL, and incubated at 37°C for one to three hours.

The digestion treatment with a methylation sensitive restriction enzyme may be a digestion treatment comprising First (A) step of mixing a single-stranded DNA (plus strand) containing an objective DNA region with a masking oligonucleotide comprising a nucleotide sequence complementary to a nucleotide sequence of a recognition site of the methylation sensitive restriction enzyme, thereby generating single-stranded DNA in which the recognition site of the methylation sensitive restriction enzyme is protected, and First (B) step of digesting the single-stranded DNA selected in First (A) step with the methylation sensitive restriction enzyme.

The term "masking oligonucleotide" used herein means oligonucleotide having a nucleotide sequence complementary to the nucleotide sequence of the recognition site of the methylation sensitive restriction enzyme, and is oligonucleotide that forms double strand by complementary base-pairing at least one site (even every site is possible) of several recognition sites of the methylation sensitive restriction enzyme contained in the objective DNA region in the single-stranded DNA (that is, the site is made into double-stranded state), thereby enabling the methylation sensitive restriction enzyme that uses only double-stranded DNA as a substrate to digest the site, and improving digestion efficiency at the site for the methylation sensitive restriction enzyme capable of digesting single-stranded DNA (methylation sensitive restriction enzyme capable of digesting single-stranded DNA also digests double-stranded DNA, and digestion efficiency thereof is higher with respect to double-stranded DNA than with respect to single-stranded DNA), and means oligonucleotide not inhibiting formation of double strand between single-stranded DNA containing the objective DNA region and single-stranded immobilized oligonucleotide. Further, when a sample is a single-stranded DNA, the masking oligonucleotide should be oligonucleotide that is unavailable in a reaction for extending an extension primer by using a later-described reverse primer (plus strand) as the extension primer and the masking oligonucleotide (minus strand) as a template. As a nucleotide length, 8 to 200 bases long is preferred.

The masking oligonucleotide to be mixed with a DNA sample derived from genomic DNA may be one kind or plural kinds. When plural kinds are used, many of recognition sites of the methylation sensitive restriction enzyme in the single-stranded DNA containing the objective DNA region become double-strand state, and "DNA remaining undigested" as will be described later by the methylation sensitive restriction enzyme can be minimized. For example, it is particularly useful to use the masking oligonucleotide designed in accordance with a site intended not to be digested when it is methylated and intended to be digested when it is not methylated among several recognition sequences of the methylation sensitive restriction enzyme contained in the objective DNA region (for example, the site that is methylated at 100% in a diseased patient specimen, but is not methylated at 100% in a healthy specimen).

As a concern in a digestion treatment in First step, a fear that a recognition sequence containing non-methylated cytosine cannot be completely digested (so called "DNA remaining undigested") can be recited. When such a fear is problematic, since the "DNA remaining undigested" can be minimized if recognition sites of the methylation sensitive restriction enzyme abundantly exist, it is considered that as the objective DNA region, the one having one or more recognition sites of the methylation sensitive restriction enzyme is preferred and the more the better.

In the present invention or a methylation rate measuring method as will be described later, one preferable embodiment is that "a DNA sample derived from a genomic DNA contained in a biological specimen" is a DNA sample digested in advance with a restriction enzyme recognition cleavage site for which in not present in the objective DNA region possessed by the genomic DNA. Here, when a digested substance of a genomic DNA contained in a biological specimen is selected with the use of present immobilized oligonucleotide, shorter template DNA is more likely to be selected, and when the objective region is amplified by PCR, shorter template DNA is more preferred. Therefore, a digestion treatment may be executed while using a restriction enzyme whose recognition cleavage site excludes the objective DNA region directly on the DNA sample derived from a genomic DNA contained in a biological specimen. As a method of digesting with a restriction enzyme recognition cleavage site for which is not present in the objective DNA region, a commonly used restriction enzyme treatment method may be used. These embodiments are preferred because the methylation amount can be determined accurately by digesting the biological specimen itself in advance with a restriction enzyme as described above. Such a method is useful for avoiding the "DNA remaining undigested" as described above.

As a method of digesting a sample derived from a genomic DNA contained in a biological specimen with the methylation sensitive restriction enzyme, when the biological specimen is a genomic DNA itself, the method similar to that described above is preferred, and when the biological specimen is a tissue lysate, a cell lysate or the like, a digestion treatment may be executed using a large excess of methylation sensitive restriction enzyme, for example, a methylation sensitive restriction enzyme in an amount of 500 times (10 U) or more with respect to 25 ng of the DNA amount, according to a similar method as described above.

Basically, genomic DNA exists as double-stranded DNA. Therefore, in the present operation, not only a methylation sensitive restriction enzyme (for example, HhaI) capable of digesting single-stranded DNA, but also a methylation sensitive restriction enzyme capable of digesting double-stranded DNA (for example, HpaII, BstUI, NarI, SacII, HhaI and the like) may be used.

In Second step, single-stranded DNA (plus strand) containing the objective DNA region from the DNA sample subjected to the digestion treatment obtained in First step is acquired, and the single-stranded DNA is selected by causing base-pairing between the single-stranded DNA (plus strand) and a single-stranded immobilized oligonucleotide comprising a nucleotide sequence complementary to a part (provided that, not containing the objective DNA region) of 3'-end of the single-stranded DNA.

"Single-stranded immobilized oligonucleotide" is a single-stranded immobilized oligonucleotide comprising a nucleotide sequence complementary to a part (provided that, not containing the objective DNA region) of 3'-end of the single-stranded DNA (plus strand) containing the objective DNA region (hereinafter, also referred to as "present immobilized oligonucleotide").

The present immobilized oligonucleotide is used for selecting single-stranded DNA (plus strand) containing an objective DNA region from a DNA sample derived from genomic DNA contained in a biological specimen. The present immobilized oligonucleotide is preferably 5 to 50 bases long.

5'-end side of the present immobilized oligonucleotide may be immobilized to a carrier, and 3'-end side thereof may be a free state to allow one extension reaction proceeding from 5'-end toward 3'-end by Third step and Step A2 as will be described later.

Alternatively, the present immobilized oligonucleotide may be immobilized to a carrier at its 5'- or 3'-end.

As to the expression "the one that can be immobilized to a carrier", it suffices that the present immobilized oligonucleotide is immobilized to a carrier when the single-stranded DNA (plus strand) containing the objective DNA region is selected, and (1) it may be immobilized by binding between the present immobilized oligonucleotide and the carrier before base-pairing between the single-stranded DNA (plus chain) and the present immobilized oligonucleotide, or (2) it may be immobilized by binding between the present immobilized oligonucleotide and the carrier after base-pairing between the single-stranded DNA (plus chain) and the present immobilized oligonucleotide.

For obtaining such a structure, the 5'-end of the oligonucleotide having the nucleotide sequence complementary to a part (provided that not containing the objective DNA region) of the 3'-end of the single-stranded DNA (plus strand) containing the objective DNA region (hereinafter, also referred to "present oligonucleotide") may be immobilized to a carrier according to a general genetic engineering method or commercially available kit, apparatus and the like (binding to solid phase). A concrete exemplary method involves biotinylating the 5'-end of the present oligonucleotide, and immobilizing the obtained biotinylated oligonucleotide to a support coated with streptavidin (for example, a PCR tube coated with streptavidin, magnetic beads coated with streptavidin and so on).

Also, such a method can be recited that after covalently binding a molecule having an active functional group such as an amino group, an aldehyde group or a thiol group to 5'-end side of the present oligonucleotide, the product is covalently bound to a support made of glass, silica or heat-resistant plastic having a surface activated with a silane coupling agent or the like, via a spacer, a cross linker or the like such as five serially-connected triglycerides. Also, a method of chemically synthesizing from the 5'-end side of the present oligonucleotide directly on a support made of glass or silicon is recited.

In Second step, concretely, in the case where the present immobilized oligonucleotide is a biotinated oligonucleotide, for example,
(a) first, by adding an annealing buffer and a biotinated oligonucleotide (currently in a free state because immobilization is conducted by binding between the present immobilized oligonucleotide and the carrier after base-pairing between the single-stranded DNA (plus strand) and the present immobilized oligonucleotide) to a DNA sample derived from genomic DNA contained in a biological specimen, a mixture is obtained. Then the obtained mixture is heated at 95°C for several minutes to make the double-stranded DNA containing the objective DNA region existing in the DNA sample derived from genomic DNA contained in the biological specimen into a single strand. Then the mixture is rapidly cooled to a temperature lower than Tm value of biotinated oligonucleotide by about 10 to 20°C and retained at this temperature for several minutes to allow formation of a double strand with the biotinated oligonucleotide;
(b) then the temperature is recovered to room temperature; and
(c) a carrier covered with streptavidin is added with the mixture obtained in the above (b) and the mixture is retained for several minutes at 37°C to immobilize the biotinated oligonucleotide to the carrier covered with streptavidin.

As described above, in the above (a) to (c), base-pairing between the single-stranded DNA (plus strand) containing the objective DNA region and the biotinated oligonucleotide is conducted prior to immobilization of the biotinated oligonucleotide to the carrier covered with streptavidin, however, the order of these processes may be inversed. That is, for example, a mixture may be obtained by adding a DNA sample derived from genomic DNA contained in a biological specimen to a biotinated oligonucleotide immobilized to a carrier covered with streptavidin, and the obtained mixture may be heated at 95°C for several minutes to make the double-stranded DNA containing the objective DNA region existing in the DNA sample derived from genomic DNA contained in the biological specimen into a single strand, and then the mixture may be rapidly cooled to a temperature lower than Tm value of the biotinated oligonucleotide by about 10 to 20°C and retained at this temperature for several minutes to allow formation of a double strand with biotinated oligonucleotide.
(d) After immobilizing the biotinylated oligonucleotide to the support coated with streptavidin in this manner, removal and washing of the remaining solution (DNA purification) are conducted.

More concretely, for example, when a PCR tube coated with streptavidin is used, after removing the solution by pipetting or decantation first, TE buffer of a volume approximately equal to the volume of the biological specimen is added, and thereafter, TE buffer may be removed by pipetting or decantation. When magnetic beads coated with streptavidin are used, after immobilizing the beads with a magnet, the solution is removed by pipetting or decantation first, and TE buffer of a volume approximately equal to the volume of the biological specimen is added, and thereafter, TE buffer may be removed by pipetting or decantation.

Then, by executing these operations several times, the remaining solution is removed and washed (DNA purification).

These operations are important for removing unimmobilized DNA, or DNA floating in the solution digested with the restriction enzyme as will be described later, from the reaction solution. If these operations are inadequate, the DNA floating in the reaction solution will be a template and an unexpected amplification product will be obtained by an amplification reaction. In order to avoid non-specific binding between the support and DNA in the biological specimen, the above operations may be executed while a large amount of DNA having a nucleotide sequence which is completely different from that of the objective region (for example, rat DNA and so on, in the case of a human biological specimen) is added to the biological specimen.

As a preferred aspect in Second step, in base-pairing between single-stranded DNA (plus strand) containing the objective DNA region and single-stranded immobilized oligonucleotide having a nucleotide sequence complementary to a part (provided that, not containing the objective DNA region) of the 3'-end of the single-stranded DNA, base-pairing in a reaction system containing a divalent cation can be recited. More preferably, the divalent cation is a magnesium ion. The "reaction system containing a divalent cation" used herein means a reaction system that contains a divalent cation in an annealing buffer used for base-pairing between the single-stranded DNA (plus strand) and the single-stranded immobilized oligonucleotide, and concretely and preferably contains a salt containing magnesium ions (for example, MgOAc₂, MgCl₂ and so on) in a concentration of 1 mM to 600 mM.

In Third step, double-stranded DNA is extensionally-formed from the single-stranded DNA by allowing one extension of a primer using the single-stranded DNA selected in Second step as a template and the single-stranded immobilized oligonucleotide as the primer. In this case, extension reaction may be conducted using DNA polymerase for allowing one extension of the primer using the single-stranded DNA as a template and the single-stranded immobilized oligonucleotide as a primer.

Concretely, Third step may be conducted, for example, in the following manner when the present immobilized oligonucleotide is a biotinated oligonucleotide.

Single-stranded DNA containing the objective DNA region selected in Second step is added with 17.85 µL of sterilized ultrapure water, 3 µL of optimum 10 × buffer (100 mM Tris-HCl pH 8.3, 500 mM KCl, 15 mM MgCl₂), 3 µL of 2 mM dNTP, and 6 µL of 5N betaine, and then the mixture is added with 0.15 µL of AmpliTaq (a kind of DNA polymerase: 5 U/µL) to make the liquid amount 30 µL, and incubated at 37°C for 2 hours. After removing the incubated solution by pipetting or decantation, TE buffer of a volume approximately equal to the volume of the biological specimen may be added, and the TE buffer may be removed by pipetting or decantation.

More concretely, for example, when a PCR tube coated with streptavidin is used, after removing the solution by pipetting or decantation first, TE buffer of a volume approximately equal to the volume of the biological specimen is added, and thereafter, TE buffer may be removed by pipetting or decantation. When magnetic beads coated with streptavidin are used, after immobilizing the beads with a magnet, the solution is removed by pipetting or decantation first, and TE buffer of a volume approximately equal to the volume of the biological specimen is added, and thereafter, TE buffer may be removed by pipetting or decantation.

Third step may be a step comprising a step of separating the single-stranded DNA selected in Second step from the immobilized oligonucleotide and temporarily separating the single-stranded DNA into a single-stranded state, and extensionally-forming a double-stranded DNA from the single-stranded DNA by allowing one extension of a primer using the generated DNA in a single-stranded state (plus strand) as a template and a forward primer comprising a nucleotide sequence (minus strand) complementary to a partial nucleotide sequence (plus strand) located on further 3'-end side than the 3'-end of the nucleotide sequence (plus strand) of the objective DNA region as an extension primer.

As a method in this case, concretely, heating at 95°C for several minutes may be conducted for making the double-stranded DNA into a single strand. Further, after adding a forward primer, the reaction is rapidly cooled to a temperature lower than Tm value of the forward primer by about 10 to 20°C and retained at this temperature for several minutes to allow formation of a double strand between the generated DNA in a single-stranded state (plus strand) and the forward primer. The generated solution of double-stranded DNA is added with 3 µL of optimum 10 × buffer (100 mM Tris-HCl pH 8.3, 500 mM KCl, 15 mM MgCl₂), 3 µL of 2 mM dNTP, and 6 µL of 5N betaine, and then the mixture is added with 0.15 µL of AmpliTaq (a kind of DNA polymerase: 5 U/µL) and added with sterilized ultrapure water to make the liquid amount 30 µL, and incubated at 37°C for 2 hours.

Third step may be conducted independently of Fourth step, or may be conducted sequentially with the PCR reaction conducted in Fourth step.

Fourth step comprises, as a pre step of the following regular steps, a step (pre step) of temporarily separating the extensionally-formed double-stranded DNA obtained in Third step (extensionally-formed double-stranded DNA not containing an unmethylated CpG pair in the recognition site of the methylation sensitive restriction enzyme) into a single-stranded state, and as regular steps:
(a) Step A (regular step) comprising Step A1 of selecting the DNA in a single-stranded state by causing base-pairing between the generated DNA in a single-stranded state (plus strand) and the single-stranded immobilized oligonucleotide (minus strand), and Step A2 of extensionally-forming double-stranded DNA from the DNA in a single-stranded state by causing one extension of a primer by using the DNA in a single-stranded state selected in Step A1 as a template and the single-stranded immobilized oligonucleotide as the primer, and
(b) Step B (regular step) of extensionally-forming double-stranded DNA from the DNA in a single-stranded state by causing one extension of an extension primer by using the generated DNA in a single-stranded state (minus strand) as a template, and a primer (reverse primer) comprising a nucleotide sequence (plus strand) complementary to a partial nucleotide sequence (minus strand) of the nucleotide sequence of the DNA in a single-stranded state (minus strand), wherein the partial nucleotide sequence (minus strand) is located on further 3'-end side than the 3'-end of the nucleotide sequence (minus strand) complementary to the nucleotide sequence (plus strand) of the objective DNA region, as the extension primer,
wherein the methylated DNA in the objective DNA region is amplified to a detectable level by repeating each regular step after temporarily separating the extensionally-formed double-stranded DNA obtained in the regular steps into a single-stranded state, and an amount of the amplified DNA is quantified.

In Forth step, as a pre step of the following regular steps, the extensionally-formed double-stranded DNA which is an undigested substance obtained in Third step (extensionally-formed double-stranded DNA not containing an unmethylated CpG pair in the recognition site of the methylation sensitive restriction enzyme) is temporarily separated into a single-stranded state. Concretely, for example, the extensionally-formed double-stranded DNA which is an undigested substance obtained in Third step (extensionally-formed double-stranded DNA not containing an unmethylated CpG pair in the recognition site of the methylation sensitive restriction enzyme) is added with an annealing buffer to obtain a mixture.

Then, the obtained mixture is heated at 95°C for several minutes. Then, the following regular steps are conducted.
(i) The generated single-stranded DNA (plus strand) is rapidly cooled to the temperature lower by about 10 to 20°C than Tm of single-stranded immobilized oligonucleotide (minus strand), and kept at this temperature for several minutes for allowing annealing with the single-stranded immobilized oligonucleotide (minus strand).
(ii) Thereafter, the temperature is restored to room temperature (Step A1 in Step A).
(iii) Double-stranded DNA is extensionally formed from the single-stranded DNA by a single extension of a primer by using the single-stranded DNA selected in the above (i) as a template and the single-stranded immobilized oligonucleotide as the primer (that is, Step A2 in Step A). Concretely, for example, it may be practiced in accordance with the operation method or the like in the extension reaction as will be described later or in Second step of the present invention as described above.
(iv) Double-stranded DNA is extensionally formed from the single-stranded DNA by allowing one extension of an extension primer by using the generated single-stranded DNA (minus strand) as a template, and the extension primer (reverse primer) having a nucleotide sequence (plus strand) complementary to the partial nucleotide sequence (minus strand) of a nucleotide sequence possessed by the single-stranded DNA (minus strand) and the partial nucleotide sequence (minus strand) located on further 3'-end side than the 3'-end of the nucleotide sequence (minus strand) complementary to the nucleotide sequence (plus strand) of the objective DNA region as an extension primer (that is, Step B). Concretely, for example, similarly to the above (iii), it may be practiced in accordance with the operation method or the like in the extension reaction as will be described later or in Second step of the present invention as described above.
(v) Methylated DNA in the objective DNA region is amplified to a detectable level by repeating each regular step in Fourth step (for example Step A and Step B) after temporarily separating the extensionally-formed double-stranded DNA obtained in each regular step into a single strand state, and the amplified DNA is quantified. Concretely, for example, similarly to the above description, it may be practiced in accordance with the operation method or the like as will be described later or in pre step, Step A and Step B in Forth step of the present invention as described above.

As a method of amplifying the objective DNA region (that is, objective region) after a digestion treatment with a methylation sensitive restriction enzyme, for example, PCR may be used. Since a immobilized oligonucleotide can be used as one of the primers in amplifying the objective region, an amplification product can be obtained by conducting PCR while adding only the other of the primers, and its amplification product is also immobilized. At this time, by using a primer labeled in advance with fluorescence or the like and utilizing the label as an index, it is possible to evaluate presence or absence of the amplification product without executing a burdensome operation such as electrophoresis. As a PCR reaction solution, for example, a reaction solution prepared by mixing the DNA obtained in Third step of the present invention with 0.15 µL of a 50 µM primer solution, 2.5 µL of 2 mM dNTP, 2.5 µL of a buffer (100 mM Tris-HCl pH 8.3, 500 mM KCl, 20 mM MgCl₂, 0.01% Gelatin), and 0.2 µL of AmpliTaq Gold (one kind of thermostable DNA polymerase: 5 U/µL), and adding sterilized ultrapure water to make the liquid volume 25 µL can be recited.

Since an objective DNA region (namely, an objective region) often has a GC rich nucleotide sequence, the reaction may sometimes be executed while adding an appropriate amount of betaine, DMSO or the like. In one exemplary reaction conditions, the reaction solution as described above is retained at 95°C for 10 minutes, and then a cycle made up of 30 seconds at 95°C, 30 seconds at 55 to 65°C, and 30 seconds at 72°C is repeated 30 to 40 cycles. After conducting such PCR, the obtained amplification product is detected. For example, when a primer labeled in advance is used, after executing washing and purification operations similar to those as described above, an amount of an immobilized fluorescent label may be measured. When PCR is conducted using a normal primer that is not labeled, a probe or the like that is labeled with gold colloid particles, fluorescence or the like is caused to anneal, and detection may be achieved by measuring an amount of the probe bound to the objective region. Also, in order to determine an amount of the amplification product more accurately, for example, a real-time PCR method may be used. The real-time PCR is a method in which PCR is monitored in real time, and the obtained monitor result is analyzed kinetically, and is known as a high-accuracy quantitative PCR method capable of detecting a very small difference as small as twice the gene amount. As such a real-time PCR method, for example, a method using a probe such as a template-dependent nucleic acid polymerase probe, a method of using an intercalator such as SYBR-Green and the like can be recited. As an apparatus and a kit for the real-time PCR method, those commercially available may be used. As described above, detection may be executed by any method well-known heretofore without any particular limitation. Such methods make it possible to conduct the operations up to detection without changing the reaction container.

Further, the objective region can be amplified by using a biotinylated oligonucleotide having the same nucleotide sequence as the immobilized oligonucleotide as one of the primers, or biotinylated oligonucleotide newly designed on the 3'-end side from the immobilized oligonucleotide as one of the primers, and a primer of a complementary side. In this case, since the obtained amplification product is immobilized if there is a support coated with streptavidin, for example, when PCR is executed in a PCR tube coated with streptavidin, use of labeled primer as described above will facilitate the detection of the amplification product because it will be immobilized inside the tube. When the foregoing immobilized oligonucleotide is immobilized by covalent bonding or the like, the solution containing an amplification product obtained by PCR may be transferred to a container where a support coated with streptavidin is present, and the amplification product may be immobilized. The detection may be executed in the manner as described above. The complementary side primer for amplifying an objective region should be a primer that is capable of amplifying an objective region having one or more recognition sites of a methylation sensitive restriction enzyme and not containing the recognition site. The reason is as follows. When only the most 3'-end side recognition site of the methylation sensitive restriction enzyme of a DNA chain (newly-generated strand) on the immobilized oligonucleotide side of the double-stranded DNA obtained by selection and one extension reaction is not methylated, only that part will be digested by the methylation sensitive restriction enzyme. Even if washing operation is conducted as described above after digestion, double-stranded DNA in which a part of the 3'-end in newly-generated strand is lost remains immobilized. When the complementary side primer contains the most 3'-end side recognition site of the methylation sensitive restriction enzyme, several bases on the 3'-end side of the primer anneal with several bases on the 3'-end side of the newly-generated strand, so that the objective region can be amplified by PCR.

The present invention also provides a modified method further comprising a step of adding into a reaction system single-stranded oligonucleotide (minus strand) having a nucleotide sequence complementary to a part (provided that, not containing the objective DNA region) of the 3'-end of single-stranded DNA containing an objective DNA region (plus strand) and is in a free state (Additional pre step), before or after pre step in Fourth step of the present invention.
That is,

### (Modified method 1)

A method further comprising prior to the pre step in Fourth step of the present invention:
a step (Additional pre step) of adding into the reaction system a single-stranded oligonucleotide (minus strand) in a free state comprising a nucleotide sequence complementary to a part (provided that, not containing the objective DNA region) of 3'-end of the single-stranded DNA (plus strand) containing the objective DNA region, and further comprising the following one step as each regular step in Fourth step of the present invention: (c) Step C (regular step) comprising:
   (i) Step C1 of selecting the DNA in a single-stranded state by base-pairing between the generated DNA in a single-stranded state (plus strand) and the single-stranded oligonucleotide (minus strand) added into the reaction system in Additional pre step, and
   (ii) Step C2 of extensionally-forming double-stranded DNA from the DNA in a single-stranded state by allowing one extension of a primer by using the DNA in a single-stranded state selected in Step C1 as a template and the single-stranded oligonucleotide (minus strand) as the primer.

### (Modified method 2)

A method further comprising after the pre step in Fourth step of the present invention:
a step (Additional pre step) of adding into the reaction system a single-stranded oligonucleotide (minus strand) in a free state comprising a nucleotide sequence complementary to a part (provided that, not containing the objective DNA region) of 3'-end of the single-stranded DNA (plus strand) containing the objective DNA region, and
a step (Additional re-pre step) of temporarily separating the extensionally-formed double-stranded DNA which is an undigested substance obtained through Third step and the Additional pre step (extensionally-formed double-stranded DNA not containing an unmethylated CpG pair in the recognition site of the methylation sensitive restriction enzyme) into a single-stranded state, and further comprising the following one step as each regular step in Fourth step in the present invention (hereinafter, also referred to as the present methylation rate measuring method):
   (c) Step C (Regular step) comprising:
      (i) Step C1 of selecting the DNA in a single-stranded state by base-pairing between the generated DNA in a single-stranded state (plus strand) and the single-stranded oligonucleotide (minus strand) added into the reaction system in Additional pre step, and
      (ii) Step C2 of extensionally-forming double-stranded DNA from the DNA in a single-stranded state by allowing one extension of a primer by using the DNA in a single-stranded state selected in Step C1 as a template and the single-stranded oligonucleotide (minus strand) as the primer.

In the modified methods, it is possible to readily improve the amplification efficiency of the objective DNA region in Fourth step by, for example, adding "single-stranded oligonucleotide (minus strand) having a nucleotide sequence complementary to a part (not containing the objective DNA region) of the 3'-end of single-stranded DNA (plus strand) containing an objective DNA region and is in a free state" to a reaction system externally. The single-stranded oligonucleotide (minus strand) added into the reaction system in Additional pre step may have the same nucleotide sequence as that of the single-stranded immobilized oligonucleotide, or may have a shorter nucleotide sequence or a longer nucleotide sequence insofar as it is single-stranded oligonucleotide having a nucleotide sequence complementary to a part (not containing the objective DNA region) of the 3'-end of single-stranded DNA and has the 5'-end which is as same as the single-stranded immobilized oligonucleotide and in a free state. However, in the case of a nucleotide sequence longer than the single-stranded immobilized oligonucleotide, it is necessary to be single-stranded oligonucleotide in a free state which is unavailable in reaction of extending an extension primer by using the reverse primer (plus strand) as an extension primer, and the single-stranded oligonucleotide (minus strand) as a template.

While an explanation was made for the case where PCR is executed while using a immobilized oligonucleotide as one of primers and adding the other of primers in amplifying the objective region, when other methods for detecting the objective product (for example, an analytical method capable of comparing the amount of each amplification product obtained by PCR) are executed, PCR may be executed while adding a pair of primers rather than using immobilized oligonucleotide as either one of primers in amplifying the objective region as described above. After conducting such PCR, an amount of the obtained amplification product is determined.

Fourth step has repeated steps, and for example, "generated single-stranded DNA (plus strand)" in Step A1 means "generated "free" DNA in a single-stranded state (plus strand)" both in first operation of Fourth step and in second or later repeated operation of Fourth step.

In Step B, "generated single-stranded DNA (minus strand)" means "generated "immobilized" DNA in a single-stranded state (plus strand)" both in first operation of Fourth step and in second or later repeated operation of Fourth step. However, when Fourth step further has Step C additionally, it means "generated "immobilized" DNA in a single-stranded state (plus strand)" in first operation of Fourth step, while it means both "generated "immobilized" DNA in a single-stranded state (plus strand)" and "generated "free" DNA in a single-stranded state (plus strand)" in second or later repeated operation of Fourth step.

In Step A, "extensionally-formed double-stranded DNA" obtained in each regular step of Fourth step means "extensionally-formed double-stranded DNA not containing an unmethylated CpG pair in the recognition site of the methylation sensitive restriction enzyme" in the first operation of Fourth step, while it means both "extensionally-formed double-stranded DNA not containing an unmethylated CpG pair in the recognition site of the methylation sensitive restriction enzyme" and "extensionally-formed double-stranded DNA containing an unmethylated CpG pair in the recognition site of the methylation sensitive restriction enzyme" in the second or later repeated operation of Fourth step. In Step B, it means "extensionally-formed double-stranded DNA in which a CpG pair is unmethylated in every recognition site of the methylation sensitive restriction enzyme" both in the first operation of Fourth step and in the second or later repeated operation of Fourth step.

The same applies to a case where Fourth step further has Step C additionally.

In a case where Fourth step further has Step C additionally, "generated single-stranded DNA (plus strand)" in Step C1 means "generated "free" single-stranded DNA (plus strand)" both in the first operation of Fourth step and in the second or later repeated operation of Fourth step.

The present invention also provides a method of measuring methylation rate (that is, the present methylation rate measuring method) further comprising the following two steps as steps of the present invention:
(5) Fifth step of amplifying DNA (total amount of methylated DNA and non-methylated DNA) of the objective DNA region to a detectable level by conducting Fourth step of the present invention (including the above modified methods) without conducting Third step of the present invention (including the above modified methods) after conducting First step and Second step of the present invention (including the above modified methods), and quantifying the amplified DNA; and
(6) Sixth step of calculating a rate of methylated DNA in the objective DNA region based on a difference obtained by comparing the DNA amount quantified by Fourth step of the present invention (including the above modified methods) and the DNA amount quantified in Fifth step.

The methylation rate measuring method may be used in the following situations.

It is known that DNA methylation abnormality occurs in various diseases (for example, cancer), and it is believed that the degree of various diseases can be measured by detecting this DNA methylation abnormality.

For example, when there is a DNA region where methylation occurs at 100% in a genomic DNA contained in a specimen derived from a diseased organism, and the present invention or the present methylation rate measuring method is executed for the DNA region, the amount of methylated DNA would increase, and for example, when there is a DNA region where methylation does not occur at 100% in a genomic DNA contained in a specimen derived from a diseased organism, and the present invention or the present methylation rate measuring method is executed for the DNA region, the amount of methylated DNA would be approximately 0. For example, when there is a DNA region where the methylation rate is low in a genomic DNA contained in a specimen derived from a healthy organism, and a DNA region where the methylation rate is high in a genomic DNA contained in a specimen derived from a diseased organism, and the present invention or the present methylation rate measuring method is executed for the DNA region, the amount of methylated DNA would be approximately 0 for a healthy subject, and a significantly higher value than that of a healthy subject would be exhibited by a disease subject, so that the "degree of disease" can be determined based on this difference in value. The "degree of disease" used herein has the same meaning as those commonly used in this field of art, and concretely means, for example, malignancy when the biological specimen is a cell, and means, for example, abundance of disease cells in the tissue when the biological specimen is a tissue. Further, when the biological specimen is plasma or serum, it means the probability that the individual has a disease. Therefore, the present invention or the present methylation rate measuring method makes it possible to diagnose various diseases by examining methylation abnormality.

Restriction enzymes, primers or probes that can be used in various methods for measuring a methylated DNA amount in an objective region, and for measuring a methylation rate in the present invention or the present methylation rate measuring method are useful as reagents of a detection kit. The present invention also provides a detection kit containing these restriction enzymes, primers or probes as reagents, and a detection chip in which these primers or probes are immobilized on a carrier, and a scope of the present invention or the present methylation rate measuring method includes use in the form of the detection kit or the detection chip as described above utilizing the substantial principle of such a method.

### Examples

In the following, the present invention will be described in detail by way of examples, however, the present invention will not be limited to these examples.

### Example 1

Methylated oligonucleotide GPR7-2079-2176/98 mer-M(7) consisting of the nucleotide sequence of SEQ ID NO:17 in which the recognition site of HpaII is methylated, and unmethylated oligonucleotide GPR7-2079-2176/98 mer-UM consisting of the nucleotide sequence of SEQ ID NO:2 in which the recognition site of HpaII is not methylated were synthesized, and 0.001 pmol/10 µL TE buffer solutions were prepared respectively.

### <Methylated oligonucleotide in which the recognition site of HpaII is methylated>

N represents methylated cytosine.

GPR7-2079-2176/98mer-M(7):

5′-GTTGGCCACTGCGGAGTCGNGCNGGGTGGCNGGCCGCACCTACAGNGCCGNGNGNGCGG
TGAGCCTGGCCGTGTGGGGGATCGTCACACTCGTCGTGC-3′ (SEQ ID NO:17)

### <Unmethylated oligonucleotide in which the recognition site of HpaII is not methylated>

GPR7-2079-2176/98 mer-UM:

5′-GTTGGCCACTGCGGAGTCGCGCCGGGTGGCCGGCCGCACCTACAGCGCCGCGCGCGCGG
TGAGCCTGGCCGTGTGGGGGATCGTCACACTCGTCGTGC-3′ (SEQ ID NO:18)

Group A (no treatment group): The oligonucleotide solution prepared as described above was added with 5 µL of a 10xbuffer suited for HpaII and HhaI (330 mM Tris-Acetate pH 7.9, 660 mM KOAc, 100 mM MgOAc₂, 5 mM Dithiothreitol) and 5 µL of 10×BSA (Bovine serum albumin 1mg/ml), and the resultant mixture was added with sterilized ultrapure water to make the liquid volume 50 µL.

Group B (HpaII digestion treatment group): The oligonucleotide solution prepared as described above was added with 12U of HpaII, 5 µL of 10xbuffer suited for HpaII and HhaI (330 mM Tris-Acetate pH 7. 9, 660 mM KOAc, 100 mM MgOAc₂, 5 mM Dithiothreitol), and 5 µL of 10×BSA (Bovine serum albumin 1mg/ml), and the resultant mixture was added with sterilized ultrapure water to make the liquid volume 50 µL.

Group C (Masking oligonucleotide addition and HpaII digestion treatment group): The oligonucleotide solution prepared as described above was added with 12U of HpaII, 5 µL of a 10xbuffer suited for HpaII and HhaI (330 mM Tris-Acetate pH 7.9, 660 mM KOAc, 100 mM MgOAc2, 5 mM Dithiothreitol), 5 µL of 10xBSA (Bovine serum albumin 1mg/ml), and 5 pmol of masking oligonucleotide MA having the nucleotide sequence of SEQ ID NO:19, and the resultant mixture was added with sterilized ultrapure water to make the liquid volume 50 µL.

### <Masking oligonucleotide>

MA: 5'-GCCACCCGGCGCGA-3' (SEQ ID NO:19)

Each reaction solution was incubated at 37°C for 18.5 hours.

Also, 5'-end biotin-labeled oligonucleotide Bio-GPR7-2176R consisting of the nucleotide sequence of SEQ ID NO:20 was synthesized, and a 0.1µM TE buffer solution was prepared.
<5'-end biotin-labeled oligonucleotide>
Bio-GPR7-2176R:5'-GCACGACGAGTGTGACGATC-3' (SEQ ID NO:20)

Each three of 50 µL of the obtained digestion-treated solution of either the methylated oligonucleotide or unmethylated oligonucleotide was added with 1 µL of the 5'-end biotin-labeled oligonucleotide solution, and heated at 95°C for 5 minutes. Thereafter, the mixture was rapidly cooled to 50°C, and kept at that temperature for 5 minutes. Then, after incubation at 37°C for 5 minutes, the temperature was restored to room temperature.

Next, the previously prepared mixture was added to a PCR tube coated with streptavidin as described above, and kept at 37°C for 5 minutes.

Then, after removing the solution from the PCR tube, 100 µL of a washing buffer [0.05% Tween20-containing phosphate buffer (1 mM KH₂PO₄, 3 mM Na₂HPO·7H₂O, 154 mM NaCl pH7.4)] was added, and the buffer was removed by pipetting. This operation was repeated another two times.

Next, in the above PCR tube, PCR was conducted after adding a solution of the primer consisting of the nucleotide sequence of SEQ ID NO:21 and a solution of the primer consisting of the nucleotide sequence of SEQ ID NO: 22 (PF1 and PR1) and the following reaction condition, and methylated DNA in an objective DNA region (GPR7-2079-2176, SEQ ID NO.:23, also methylated cytosine is represented by C) was amplified.

### <Oligonucleotide primers designed for PCR>

Primer PF1:5'-GTTGGCCACTGCGGAGTCG-3' (SEQ ID NO:21)
Primer PR1:5'-GCACGACGAGTGTGACGATC-3' (SEQ ID NO:22)

### <Objective DNA region>

GPR7-2079-2176:

5′-GTTGGCCACTGCGGAGTCGCGCCGGGTGGCCGGCCGCACCTACAGCGCCGCGCGCGCGG
TGAGCCTGGCCGTGTGGGGGATCGTCACACTCGTCGTGC-3′ (SEQ ID NO:23)

As a reaction solution of PCR, a mixture prepared by mixing DNA which is a template with each 5 µL of solutions of the primer consisting of the nucleotide sequence of SEQ ID NO:21 and the primer consisting of the nucleotide sequence of SEQ ID NO:22 prepared to 3 µM, each 5 µL of 2 mM dNTPs, 5 µL of a buffer (100 mM Tris-HCl pH 8.3, 500 mM KCl, 15 mM MgCl₂, 0.01% Gelatin), 0.25µL of 5U/µL thermostable DNA polymerase (AmpliTaq Gold), and 10 µL of a 5N betaine aqueous solution, and adding sterilized ultrapure water to make the liquid volume 50 µL was used. The PCR was conducted in such a condition that the reaction solution was kept at 95°C for 10 minutes, followed by 20 cycles each including 30 seconds at 95°C, 30 seconds at 59°C and 45 seconds at 72°C.

After conducting PCR, DNA amplification was examined by 2% agarose gel electrophoresis. The result is shown in Fig. 1. In A treatment group (no treatment group) and B treatment group (HpaII treatment group), the DNA amplification was observed both in methylated oligonucleotide GPR7-2079-2176/98mer-M(7)(M) in which the recognition site of HpaII is methylated and in unmethylated oligonucleotide GPR7-2079-2176/98 mer-UM(U) in which the recognition site of HpaII is not methylated, and amplified products thereof (objective DNA region:GPR7-2079-2176) were obtained. In C treatment group (masking oligonucleotide addition and HpaII digestion treatment group), the DNA amplification was observed and an amplified product thereof (objective DNA region:GPR7-2079-2176) was obtained in the case of methylated oligonucleotide GPR7-2079-2176/98mer-M(7)(M) in which the recognition site of HpaII is methylated, however, the DNA amplification was not observed and amplified product thereof was not obtained in the case of unmethylated oligonucleotide GPR7-2079-2176/98 mer-UM(U) in which the recognition site of HpaII is not methylated.

From the above description, it was confirmed that it is possible to select the single-stranded DNA containing objective DNA region, and only methylated DNA can be amplified to a detectable level without amplifying unmethylated DNA in the objective DNA region by adding masking oligonucleotide and treating with a methylation sensitive restriction enzyme.

### INDUSTRIAL APPLICABILITY

Based on the present invention, it becomes possible to provide a method of measuring or detecting methylated DNA in a simple and convenient manner, and so on.
Free Text in Sequence Listing
   SEQ ID NO:17
Designed methylated oligonucleotide for experiment
   SEQ ID NO:18
Designed unmethylated oligonucleotide for experiment
   SEQ ID NO:19
Designed oligonucleotide for experiment
   SEQ ID NO:20
Designed biotinated oligonucleotide for fixation
   SEQ ID NO:21
Designed oligonucleotide primer for PCR
   SEQ ID NO:22
Designed oligonucleotide primer for PCR
   SEQ ID NO:23
Designed oligonucleotide consisting of objective DNA domain (GP R7-2079-2176, methylated cytosin is also shown as C)

## Claims

1. A method of measuring the content of methylated DNA in an objective DNA region in genomic DNA contained in a biological specimen, comprising:
(1) First step of subjecting a DNA sample derived from genomic DNA contained in a biological specimen to a digestion treatment with a methylation-sensitive restriction enzyme;
(2) Second step of obtaining a single-stranded DNA (plus strand) containing the objective DNA region from the DNA sample that has been subjected to the digestion treatment and obtained in First step, and causing base-pairing between the single-stranded DNA (plus strand) and a single-stranded immobilized oligonucleotide comprising a nucleotide sequence complementary to a part (provided that, not containing the objective DNA region) of 3'-end of the single-stranded DNA, thereby selecting the single-stranded DNA,
(3) Third step of extensionally-forming a double-stranded DNA from the single-stranded DNA by allowing a single extension of a primer using the single-stranded DNA selected in Second step as a template and the single-stranded immobilized oligonucleotide as the primer, and
(4) Fourth step comprising, as a pre step of the following regular steps, a step (pre step) of temporarily separating the extensionally-formed double-stranded DNA obtained in Third step into a single-stranded state, and as regular steps:
(a) Step A (regular step) comprising Step A1 of selecting the DNA in a single-stranded state by causing base-pairing between the generated DNA in a single-stranded state (plus strand) and the single-stranded immobilized oligonucleotide (minus strand), and Step A2 of extensionally-forming double-stranded DNA from the DNA in a single-stranded state by causing one extension of a primer by using the DNA in a single-stranded state selected in Step A1 as a template and the single-stranded immobilized oligonucleotide as the primer, and
(b) Step B (regular step) of extensionally-forming double-stranded DNA from the DNA in a single-stranded state by causing one extension of an extension primer by using the generated DNA in a single-stranded state (minus strand) as a template, and a primer (reverse primer) comprising a nucleotide sequence (plus strand) complementary to a partial nucleotide sequence (minus strand) of the nucleotide sequence of the DNA in a single-stranded state (minus strand), wherein the partial nucleotide sequence (minus strand) is located on further 3'-end side than the 3'-end of the nucleotide sequence (minus strand) complementary to the nucleotide sequence (plus strand) of the objective DNA region, as the extension primer,
wherein the methylated DNA in the objective DNA region is amplified to a detectable level by repeating each regular step after temporarily separating the extensionally-formed double-stranded DNA obtained in the regular steps into a single-stranded state, and an amount of the amplified DNA is quantified.

2. The method according to claim 1, wherein in Second step, base-pairing is conducted in a reaction system containing a divalent cation when the single-stranded DNA containing the objective DNA region (plus strand) and a single-stranded immobilized oligonucleotide comprising a nucleotide sequence complementary to a part (provided that, not containing the objective DNA region) of the 3'-end of the single-stranded DNA are base-paired.

3. The method according to claim 2, wherein the divalent cation is a magnesium ion.

4. The method further comprising prior to the pre step in Fourth step:
a step (Additional pre step) of adding into the reaction system a single-stranded oligonucleotide (minus strand) in a free state comprising a nucleotide sequence complementary to a part (provided that, not containing the objective DNA region) of 3'-end of the single-stranded DNA (plus strand) containing the objective DNA region, and further comprising the following one step as each regular step in Fourth step:
(c) Step C (regular step) comprising:
(i) Step C1 of selecting the DNA in a single-stranded state by base-pairing between the generated DNA in a single-stranded state (plus strand) and the single-stranded oligonucleotide (minus strand) added into the reaction system in Additional pre step, and
(ii) Step C2 of extensionally-forming double-stranded DNA from the DNA in a single-stranded state by allowing one extension of a primer by using the DNA in a single-stranded state selected in Step C1 as a template and the single-stranded oligonucleotide (minus strand) as the primer.

5. The method further comprising after the pre step in Fourth step:
a step (Additional pre step) of adding into the reaction system a single-stranded oligonucleotide (minus strand) in a free state comprising a nucleotide sequence complementary to a part (provided that, not containing the objective DNA region) of 3' -end of the single-stranded DNA (plus strand) containing the objective DNA region, and
a step (Additional re-pre step) of temporarily separating the extensionally-formed double-stranded DNA which is an undigested substance obtained through Third step and the Additional pre step (extensionally-formed double-stranded DNA not containing an unmethylated CpG pair in the recognition site of the methylation sensitive restriction enzyme) into a single-stranded state, and further comprising the following one step as each regular step in Fourth step:
(c) Step C (Regular step) comprising:
(i) Step C1 of selecting the DNA in a single-stranded state by base-pairing between the generated DNA in a single-stranded state (plus strand) and the single-stranded oligonucleotide (minus strand) added into the reaction system in Additional pre step, and
(ii) Step C2 of extensionally-forming double-stranded DNA from the DNA in a single-stranded state by allowing one extension of a primer by using the DNA in a single-stranded state selected in Step C1 as a template and the single-stranded oligonucleotide (minus strand) as the primer.

6. A method of measuring a methylation rate further comprising the following two steps as steps of the method according to any one of claims 1 to 5:
(5) Fifth step of amplifying DNA (total amount of methylated DNA and unmethylated DNA) of the objective DNA region to a detectable level by conducting Second to Fourth steps in the method of any one of claims 1 to 5 without conducting First step in the method of any one of claims 1 to 5, and quantifying the amplified DNA, and
(6) Sixth step of calculating a rate of methylated DNA in the objective DNA region based on a difference obtained by comparing the DNA amount quantified by Fourth step of any one of claims 1 to 5, and the DNA amount quantified in Fifth step.

7. The method according to any one of claims 1 to 6, wherein the biological specimen is mammalian serum or plasma.

8. The method according to any one of claims 1 to 6, wherein the biological specimen is mammalian blood or bodily fluid.

9. The method according to any one of claims 1 to 6, wherein the biological specimen is a cell lysate or a tissue lysate.

10. The method according to any one of claims 1 to 9, wherein the DNA sample derived from the genomic DNA contained in the biological specimen is a DNA sample digested in advance with a restriction enzyme the recognition cleavage site for which is not present in the objective DNA region possessed by the genomic DNA.

11. The method according to any one of claims 1 to 10, wherein the DNA sample derived from genomic DNA contained in a biological specimen is a DNA sample purified in advance.

12. The method according to any one of claims 1 to 11, wherein the digestion treatment with a methylation sensitive restriction enzyme is a digestion treatment comprising First (A) step of mixing a single-stranded DNA (plus strand) containing an objective DNA region with a masking oligonucleotide comprising a nucleotide sequence complementary to a nucleotide sequence of a recognition site of the methylation sensitive restriction enzyme, thereby generating single-stranded DNA in which the recognition site of the methylation sensitive restriction enzyme is protected, and First (B) step of digesting the single-stranded DNA selected in First (A) step with the methylation sensitive restriction enzyme.

13. The method according to any one of claims 1 to 12, wherein the methylation sensitive restriction enzyme is a restriction enzyme the recognition cleavage site for which is present in the objective DNA region possessed by genomic DNA contained in a biological specimen.

14. The method according to any one of claims 1 to 13, wherein the methylation sensitive restriction enzyme is HpaII or HhaI.

15. A method of measuring the content of methylated DNA in an objective DNA region possessed by genomic DNA contained in a biological specimen, comprising:
(1) First step of subj ecting a DNA sample derived from genomic DNA contained in a biological specimen to a digestion treatment with a methylation sensitive restriction enzyme;
(2) Second step of obtaining single-stranded DNA (plus strand) containing the objective DNA region from the DNA sample that has been subjected to the digestion treatment and obtained in First step, and causing base-pairing between the single-stranded DNA (plus strand) and a single-stranded immobilized oligonucleotide comprising a nucleotide sequence complementary to a part of the single-stranded DNA, thereby selecting the single-stranded DNA,
(3) Third step comprising a step of temporarily separating the single-stranded DNA selected in Second step into a single-stranded state, and extensionally-forming double-stranded DNA from the single-stranded DNA by allowing one extension of a primer using the generated DNA in a single-stranded state (plus strand) as a template and a forward primer comprising a nucleotide sequence (minus strand) complementary to a partial nucleotide sequence (plus strand) located on further 3'-end side than the 3'-end of the nucleotide sequence (plus strand) of the objective DNA region as an extension primer,
(4) Fourth step comprising, as a pre step of each of the following regular steps, a step (pre step) of temporarily separating the extensionally-formed double-stranded DNA obtained in Third step into a single-stranded state, and as regular steps:
(a) Step A (Regular step) comprising Step A1 of selecting the DNA in a single-stranded state by causing base-pairing between the generated DNA in a single-stranded state (plus strand) and the forward primer (minus strand), and Step A2 of extensionally-forming double-stranded DNA from the DNA in a single-stranded state by causing one extension of a primer by using the DNA in a single-stranded state selected in Step A1 as a template and the forward primer as an extension primer, and
(b) Step B (Regular step) of extensionally-forming double-stranded DNA from the DNA in a single-stranded state by causing one extension of an extension primer by using the generated DNA in a single-stranded state (minus strand) as a template, and a reverse primer comprising a nucleotide sequence (plus strand) complementary to a partial nucleotide sequence (minus strand) of the nucleotide sequence of the DNA in a single-stranded state (minus strand), wherein the partial nucleotide sequence (minus strand) is located on further 3'-end side than the 3'-end of the nucleotide sequence (minus strand) complementary to the nucleotide sequence (plus strand) of the objective DNA region, as the extension primer, wherein the methylated DNA in the objective DNA region is amplified to a detectable level by repeating each regular step in Fourth step after temporarily separating the extensionally-formed double-stranded DNA obtained in the regular steps into a single-stranded state, and an amount of the amplified DNA is quantified.

16. The method according to claim 15, wherein the single-stranded immobilized oligonucleotide is a single-stranded immobilized oligonucleotide immobilized at 5'- or 3'-end.

17. The method according to claim 15 or 16, wherein in Second step, base-pairing is conducted in a reaction system containing a divalent cation when the single-stranded DNA containing the objective DNA region (plus strand) and a single-stranded immobilized oligonucleotide comprising a nucleotide sequence complementary to a part (provided that, not containing the objective DNA region) of the 3'-end of the single-stranded DNA are base-paired.

18. A method of measuring a methylation rate further comprising the following two steps as steps of the method of claim 15 or 16:
(5) Fifth step of amplifying DNA (total amount of methylated DNA and unmethylated DNA) of the obj ective DNA region to a detectable level by conducting Second to Fourth steps in the method of any one of claims 1 to 5 without conducting First step in the method of claim 15 or 16, and quantifying the amplified DNA, and
(6) Sixth step of calculating a rate of methylated DNA in the objective DNA region based on a difference obtained by comparing the DNA amount quantified in Fourth step of claim 15 or 16, and the DNA amount quantified in Fifth step.

19. The method according to claim 15 or 16, wherein the biological specimen is mammalian serum or plasma.

20. The method according to claim 15 or 16, wherein the biological specimen is mammalian blood or bodily fluid.

21. The method according to claim 15 or 16, wherein the biological specimen is a cell lysate or a tissue lysate.

22. The method according to claim 15 or 16, wherein the DNA sample derived from the genomic DNA contained in the biological specimen is a DNA sample digested in advance with a restriction enzyme the recognition cleavage site for which is not present in the objective DNA region possessed by the genomic DNA.

23. The method according to claim 15 or 16, wherein the DNA sample derived from genomic DNA contained in a biological specimen is a DNA sample purified in advance.

24. The method according to claim 15 or 16, wherein the digestion treatment with a methylation sensitive restriction enzyme is a digestion treatment comprising First (A) step of mixing a single-stranded DNA (plus strand) containing an objective DNA region with a masking oligonucleotide comprising a nucleotide sequence complementary to a nucleotide sequence of a recognition site of the methylation sensitive restriction enzyme, thereby generating single-stranded DNA in which the recognition site of the methylation sensitive restriction enzyme is protected, and First (B) step of digesting the single-stranded DNA selected in First (A) step with the methylation sensitive restriction enzyme.

25. The method according to claim 15 or 16, wherein the methylation sensitive restriction enzyme is a restriction enzyme the recognition cleavage site for which is present in the objective DNA region possessed by genomic DNA contained in a biological specimen.

26. The method according to claim 15 or 16, wherein the methylation sensitive restriction enzyme is HpaII or HhaI.


SEQUENCE LISTING

<110> Sumitomo Chemical Co., Ltd.

<120> Method for measuring DNA methylation

<130> N.112264

<140> 09724943.7
<141> 2009-03-25

<150> JP2008-077966
<151> 2008-03-25

<160> 23

<210> 1
<211> 2661
<212> DNA
<213> Homo sapiens

<400> 1
acagacatgt gccaccatgc ccagctaatt ttttgtttgt ttgtttgttt gtttgtattt 60
ttagtagaga tggggttttg ccatgttggc caggctggtc tcgaactcct gacctcgaat 120
gataatgatc cgccgcttgg cctccaaagt gctaggatta caggtgtgag ccactgcgcc 180
aggcctgggc actttcttta gtagtttgag gagcaacatt tttgacagtg tccttctgct 240
caagattcaa gatcccagat aaaattaaac catctagaga gatggcttga ttggccaaac 300
ctggatctca tgaccacttc ttgaagtggg taagtctcat aaatgctcag tccttccact 360
atgcaactga gtggggtggg tgggaagccc ctcaaaggaa aatccggttg ttcttactag 420
aaagaaaagg aaaatggatg tgaggcagtc aaaatcagca gaggtccacc acaccaccaa 480
aatgtggtga ttaaatatgg agagacagag actaacagag gtatgtgaat attgaagtat 540
gtctggacaa tagcccaatg atgagaccaa taaaatggtt accaaaatct ggttttgagt 600
agtagtgtta aatcagacca tttagtaacc attttttgtt gcaaagtttc tagcactgcc 660
caaaccctga gtggtatatg aataactcgt ccattatgta tctctttcca gtcagcataa 720
tttatccccc acctatattc ttttctgacc actcctactt ccttctcttt accaaaatct 780
aaactctaag gctgtttctt cagcaacttc tttgtttaga ttggaagata aattaaacag 840
catgcgatgt tttactgact ttcagtattt aacagaggtg atttaatttt tttttaaatc 900
caaagtcaaa cttctttata agatgaagga gaaaaatgtc ttataaaatg catatgtgaa 960
gatgccttct gagtgctttc tcatgcagac ttgttctagt ctttaatgaa tcttccttgt 1020
agacactgtg gagatgaaag atggttctcc acttctactc aaagtacaaa tcaggccggc 1080
attttgaaaa agagacaggt ttattcatag ctgcagcgtt agctggcttt gttccctgta 1140
caatttcact tttggttatt aaaatattca ctgtaggaaa taaatttgta acccatttct 1200
catattacct acacacagaa aaacaaaatt tgatatcctg gggtttattt gctgagggcg 1260
cttcccataa aagcgagaga gtgtgcgttg ggaaatgtgt ctggttaact cttttatgga 1320
taaactttag tcacaatcct cccccgcccc cctctcaccc ccagcaccct cccaacctcc 1380
cgacttcccg cctctcaagg gctggtgacc taatagcatt tttcttcgtg catattttgg 1440
cgtcgcccca tggcctggct gccttcgcct gtctgagttt tttgaaattc ctgcatgttc 1500
gccccagatt aagccagtgt gtctcaggat gtgtgttccg ttttgttctt tccccttaac 1560
gctccctgtg caacgtgtct ggggggagga gggcagggac gggagagagg gaggggcaga 1620
ggcgaggagc tgtccgcctt gcacgtttcc aatcgcatta cgtgaacaaa tagctgaggg 1680
gcggccgggc cagaacggct tgtgtaactt tgcaaacgtg ccagaaagtt taaatctctc 1740
ctccttcctt cactccagac actgcccgct ctccgggact gccgcgcggc tccccgttgc 1800
cttccaggac tgagaaaggg gaaagggaag ggtgccacgt ccgagcagcc gccttgactg 1860
gggaagggtc tgaatcccac ccttggcatt gcttggtgga gactgagata cccgtgctcc 1920
gctcgcctcc ttggttgaag atttctcctt ccctcacgtg atttgagccc cgtttttatt 1980
ttctgtgagc cacgtcctcc tcgagcgggg tcaatctggc aaaaggagtg atgcgcttcg 2040
cctggaccgt gctcctgctc gggcctttgc agctctgcgc gctagtgcac tgcgcccctc 2100
ccgccgccgg ccaacagcag cccccgcgcg agccgccggc ggctccgggc gcctggcgcc 2160
agcagatcca atgggagaac aacgggcagg tgttcagctt gctgagcctg ggctcacagt 2220
accagcctca gcgccgccgg gacccgggcg ccgccgtccc tggtgcagcc aacgcctccg 2280
cccagcagcc ccgcactccg atcctgctga tccgcgacaa ccgcaccgcc gcggcgcgaa 2340
cgcggacggc cggctcatct ggagtcaccg ctggccgccc caggcccacc gcccgtcact 2400
ggttccaagc tggctactcg acatctagag cccgcgaacc tggcgcctcg cgcgcggaga 2460
accagacagc gccgggagaa gttcctgcgc tcagtaacct gcggccgccc agccgcgtgg 2520
acggcatggt gggcgacgac ccttacaacc cctacaagta ctctgacgac aacccttatt 2580
acaactacta cgatacttat gaaaggccca gacctggggg caggtaccgg cccggatacg 2640
gcactggcta cttccagtac g 2661

<210> 2
<211> 1953
<212> DNA
<213> Homo sapiens

<400> 2
tataaattcc acgcaggcat tgaattgaat ttgttcttaa ccaaatgcgt tttatctata 60
cctggcagga atctagaagt gaaattacaa gatttatttc attttaattc tattatgaag 120
catttaatca caaataccct gaaaatgaaa agataattta tcattttacc ttgactgagc 180
aactctcctc acttcacatt catgaatcca taacgcagag aggagactgg atgattaagt 240
gtttgattag agaaaacaga ttaacctagc aaacataata aatttggctc ataagcagga 300
tggctttata aatgctcaca atacctctcc tgtataaaat catgaaccac ttcctacagt 360
gatgactcca tcgaaatagt tgagaaacat aaagcaaatg catgtttatg gctttctctt 420
tgagacatta aaagggtatt gaaaggcata tctgattcag cttataactc tggatatata 480
ttaaggaaca tgtaagaaaa tattaatgca taaaaaaagc tacaacttct caagtgttct 540
agtttccact ttgtcaataa ttacgttttc aatgtccttc tgtggactgt ttccaaaggt 600
gccaatccag acccaaagtt tcagatcact cagattcacc cttaaccttc ataacacaac 660
ccaatagctt tacgaaaaaa gttgcatatt taggtagttg ttatcccatt atgacaaaat 720
acataaaatt agcgagatat tttttagcct tcaaataagt gggaaaaaat ccttttagct 780
gagattccat ttacatcaga ataaaaatct aagttatgac taggttgaag caacgtcctg 840
tgcagcgctc cataaagttc acttagtctt caagggttcc ttacttagct aggttagtat 900
tcctggcctc tttttttagc agtgagaaaa aggatactct ccctgcccca gctttatttt 960
taaactcaca gccatatcct ggaggtctct gctggctatt tggcgcgtgg gggcggaggg 1020
gggccggggg aggggggcgg ggcggggtct ggaggtctgt gctggctatc tggcgtgtgt 1080
gtgtgtgtgt gtgtgtgtgt gtgtggttgg aggtctctgc tggctatctg gcgtgtgtgt 1140
gtgtgtggtg tggtgtgtgt aagcagtgag gttgttttag ggccagtcct tcctccgcca 1200
ctttgctgac tcaaagaccc agaggctttc ttggggtgca ggtaccatga ttccttgggc 1260
cctaagggaa tttttgttag gctagaagag tgggtgtact catgatgggt gtacccgaac 1320
attcctgggc tcaacaaaac cgattatctt tataaccgcg gcgcctagca cagcgcctgg 1380
tgccctaaac gttggctgcg ggaacgtccg agacgcgggt gcggagccgg gggcggaata 1440
actggttgcg cggcgctttg accgtaggcg ctggagcgcg tgcgttgcgt gcgcgcgcgg 1500
aggcggctgc gtcggggcgc gagaaggtgc agttccccgg cgggcgggcg ggcgggcggg 1560
cgaagctggg ctcggggcca agcgaggtct agccggagcg actgtgcccc gcctcctggg 1620
cggagcgggc ggctccccat ggtcagagcc tcgtgccggc tcggcagcgc ccggacgccg 1680
agcccagcgc gtcggccccc cggcgtgcgg gcgtctcaga gccgcggagg ggccgccggg 1740
accgtttcag cgtggcggcg ctggtgctgg cgttggccct ggaggacggc cccgagtgat 1800
ggctggcgcc tgcctcccgg gtgtctcccg ggtacagatg gagtcgtccc gcggccgccg 1860
gcggcaaggt cggcagctgc gaggccaaga gagaccccag gacacacaca gctgcctccc 1920
ggtgcgagaa gaagaccccg gcttgagagt gag 1953

<210> 3
<211> 889
<212> DNA
<213> Homo sapiens

<400> 3
cggccccatg gctccgtgtc gtgtccaagg gatgggctgg cacctcttgg accaggctta 60
ccaccagggc ccttctctga agccccagtc tgaccggcct gctgctggga atccccctct 120
gcccccacac taacctctgc tggggctgag ccagggcgcg tcggacagtc agggcgaccc 180
agccagggcg accgttggcc ccgctcctat ggggcagcag ggaccgacgt cagcagggtg 240
gggcgggcac ccgagtggta tgccccgccc tgccccgcct gcccgccctg gtggccgtct 300
gggggcgaca agtcctgaga gaaccagacg gaagcgcgct gggactgaca cgtggacttg 360
ggcggtgctg cccgggtggg tcagcctggg ctgggaggca gccccgggac acagctgtgc 420
ccacgccgtc tgagcacccc aagcccgatg cagccacccc cagacgaggc ccgcagggac 480
atggccgggg acacccagtg gtccaggtgt ggcgggggtg aggggagggg gggtgggagc 540
ggtggagatg gggccgtggg gagggagctg agatactgcc acgtgggacg atgctaggtg 600
gggagggctg agctgggcgg gctcctctgg ctgtggggcc ccctgtgttc cttgtgggag 660
gtggaaggaa gtgagtgccc tgtccttcct ccctgccatg agattccagg accggacctg 720
gcaagtgccc tatcccagcc agtgttcctg gggctcttcc aggcagggct atgttcccca 780
ggccaggggc attgtcctgg acagtcagga ggcatacccc tcgccaggtg gaaccaccct 840
gtgtatgcat gaccctgaca agcaggcgcc aggacagtca ggaggccag 889

<210> 4
<211> 863
<212> DNA
<213> Homo sapiens

<400> 4

gttgttgggt gtgaatggag aactgtgggc cctccccgac accttccagc gggacggcaa 60
cgggggccca gggggtgggc gccatcaacc ccgtcccacc gccaggacgg cgcgggggag 120
ggccggcggg ggcggggcgt cctgtaaggc gcggccccca cccgcgggcg gggcggcatt 180
cctgggaggc cggcgctctg acgtggaccc gggggccgcg ggcacggcgg gggggcggcg 240
gtccgggggc ttcttaaacc ccccgccccg gcccagcccg cacttcccga gcaccgctcc 300
gaccctggag ggagagagag ccagagagcg gccgagcgcc taggaggccc gccgagcctc 360
gccgagcccc gccagccccg gcgcgagaga agttggagag gagagcagcg cagcgcagcg 420
agtcccgtgg tcgcgcccca acagcgcccg acagcccccg atagcccaaa ccgcggccct 480
agccccggcc gcacccccag cccgcgccag catgatgaac aacagcggct actcagacgc 540
cggcctcggc ctgggcgatg agacagacga gatgccgtcc acggagaagg acctggcgga 600
ggacgcgccg tggaagaaga tccagcagaa cacattcacg cgctggtgca atgagcacct 660
caagtgcgtg ggcaagcgcc tgaccgacct gcagcgcgac ctcagcgacg ggctccggct 720
catcgcgctg ctcgaggtgc tcagccagaa gcgcatgtac cgcaagttcc atccgcgccc 780
caacttccgc caaatgaagc tggagaacgt gtccgtggcc ctcgagttcc tcgagcgcga 840
gcacatcaag ctcgtgtcca tag 863

<210> 5
<211> 2198
<212> DNA
<213> Homo sapiens

<400> 5
aagagaggca cactccctct accacaccga gggagggggc gttgagctga gaaaggttga 60
gagaatgagg gacccaggta ggtggacatc ggccaagaaa ggaaccacag cgggaggtaa 120
gaccgagagt ccccagcttg aagcgtcacc actccgggat tcccagattc caacgcgagc 180
ctggggaaag cccacagtgg agagagtccg gctggcaggg aatggcccta cccccggggt 240
gaaatctcgg agggtcgtgc agccgagtcg cgcctctgcg ctgatgcgtg agagatgccg 300
gacgtcgcgt ttgcctgtgc gagcctcgcg gatgctgtgc agtcttggtc ccctctgcgt 360
gtgtctaacg ccgaatgctg gtgtctcgag gtgtgagctt cggggccggt gtctttaaag 420
aaccaaagat tcttaaggag tgatgatctg ggtagagcgg cccgacgtag ccgcgctccc 480
aggtctcggt gcgagtcctg cggacagacc agaggagacc tgctggccag atgccccggg 540
cccaaggcgg acgccagact gtctctgcgc cagccgggct ggccttcgga atggatcagg 600
cacccgggag gccggagtgg atctcagacc ctcaagccgg gaacaaaccc gtcgatgccc 660
gtgggcctgg agtccgcctc ctccttcccg ccccacccct acccctgcct ccgaaaggct 720
tcttcgctgg tcagtagctg cgtgcccgtc tgcctgaggc tgggtcagaa ttggcgggct 780
ggtaacgacc ccgtgcacaa gcggctccca gtctctccag aaagggccga tgactaaggg 840
gtgggggtgg gggcggaggg ctggaaggtg ttagggaaga acgttagcgg cctatcctgt 900
cttcagcagc gccctctcat cttctagctc tgacgccgag cagagcagtt ggagctcggg 960
actgggaact gctggaattc ctatttagac ttctagacag tctagaaaca agaacctttc 1020
tttccctggg cctcagtttc cttgtctgta aaatcaaaag gcgggctcta ggtgtaggcc 1080
ttcttttcgc ttggtgattc tggattcctt tccttggatc cgtggggagg gggtggcagc 1140
aacagtccag ggcgttggcc gtcctgtgcc tcaagtacgt agtccccgtg cccgccccct 1200
caacaccccc agcagcccgc ccccctaagc ccgcagagca gggagctgag tgggaggggc 1260
agaggcgggg ccggttccca gtccctgctg gcggactaga gtggcgcggg ctgagcgtaa 1320
aacctgggat agccactccc ccttttcctt atccccgccc ccctgccatt ggctcccggg 1380
agaggttgac atcaaagccg cggtcttata taagccagat ccgcagggga gtccgcagaa 1440
gggttaaaca ggtctttggg cttcggcgac ctcgcccgcg gcagaaaccg gtaagaagac 1500
agtgggctgc gcgtctcatt ttcagccttg cccggactct cccaaagccg gcgcccagta 1560
gtggctccag agcccacagg tggcccccgg cagtctctgg ggcgcatgga gcggcgttaa 1620
tagggctggc ggcgcaggcc agtagccgct ccaacatgaa cctcgtgggc agctacgcac 1680
accatcacca ccatcaccac ccgcaccctg cgcaccccat gctccacgaa cccttcctct 1740
tcggtccggc ctcgcgctgt catcaggaaa ggccctactt ccagagctgg ctgctgagcc 1800
cggctgacgc tgccccggac ttccctgcgg gcgggccgcc gcccgcggcc gctgcagccg 1860
ccaccgccta tggtcctgac gccaggcctg ggcagagccc cgggcggctg gaggcgcttg 1920
gcggccgtct tggccggcgg aaaggctcag gacccaagaa ggagcggaga cgcactgaga 1980
gcattaacag cgcattcgcg gagttgcgcg agtgcatccc caacgtgccg gccgacacca 2040
agctctccaa gatcaagact ctgcgcctag ccaccagcta catcgcctac ctgatggacg 2100
tgctggccaa ggatgcacag tctggcgatc ccgaggcctt caaggctgaa ctcaagaagg 2160
cggatggcgg ccgtgagagc aagcggaaaa gggagctg 2198

<210> 6
<211> 1945
<212> DNA
<213> Homo sapiens

<400> 6
ctggatgaca gagtgagact ccgtctcaaa aaaaaagctc catttgggag gccgaggagg 60
gtggattacc tgaggtcagg agtttgagac cagcctggcc cacataggga aaccccatct 120

ctactaaaaa tacaaaaatt agtcaggtgt ggtggctgac acctataatc ccagctactt 180
gggaagctga ggcagggaga atcacttgaa ccggggaggt ggaggttgca gtgagctgag 240
atcatgccac tgcactccag cctgggcgac agggtgagat tctgtctcaa acaaacaaat 300
ttaaaagctc cgaatcctcc aaaaatacca agattttcct gtcggtaact agagatgggt 360
actgatgatt atttttaata ggtgattttc aaagatgtga acgttatcca tggagattta 420
agtctccaaa aggaaaaaaa atgcatacct ttatactaaa acttcatcac cagtcaaatt 480
tggatcatca ctaaattggc ttctacacct ctctcctaat ataaggtact tgtgtaagtt 540
tgcagttgtg agacacttat ttcctcattt ttaatgtctt ctcagtaggg ccactgatat 600
agtcactatt tgactgacca gaatggttgg cactggtgat tggctcataa agtgccctcg 660
atttaggggg ctcaattatc aaaggtttaa atcctagccc aaaccattgc tgtgatgggg 720
gttaatcaat gaaccactca gcttcacttg caaaagcggg atcacaatag ccgctttcgt 780
catgacccag cctaggtgag atttagtact taagtacact gccaggcaca caaggttaat 840
ttaacaattt aacacatttg tttcctcatc catttctcca aaccttccaa ctaatcctaa 900
cgttcgttcg gccaaatggg ccaggaattc acttaaacaa aaacaaaaaa caaaacaaac 960
aaaaaaacac tccctggggc ttggggaagg aggcaccgcc gcccatgtcg cagtctgggg 1020
gtggctcagt cctcagcacc cagatctacg gccataatgc tcttcgaggc caaggagccc 1080
ggatgcgggg cgttgccgaa ggcgtcttgc tcaggctgcg ggaaaggaga ggggtgggag 1140
cggggtgggg gcatcgcgac ccagggcaag gcggcgagtc gccgtcttcg agtcccacct 1200
gtccgaagcg gggtgagaaa aggcaaaaca tggcaaagcc atgcacctcc cagggtgggc 1260
aactcacggc cggtgaacgc cggaccctta gcagtttcca gacctttgga accggaagcg 1320
gagcctgaga gcgcgcccga gagggcgtga acgggaccgc tttcccggaa gtgcttgcgg 1380
cctctgccca gcgagctgcc ccggggtctc tctggtttcc taatcagggc aacgccgcgg 1440
gagagaacct ttaccttggc tgcactaagt tctcggtgcc actccctggc agggcgggac 1500
cttgtttagg ccctgtgatc gcgcggttcg tagtagcgca aggcgcagag tggaccttga 1560
cccgcctagg gcgggaagag tttggcccgc cgggtcccaa agggcagaat ggacgggctc 1620
ctaaatccca gggaatcctc taaattcatt gcagaaaaca gtcgggatgt gtttattgac 1680
agcggaggcg tacggagggt ggcagagctg ctgctggcca aggcggcggg gccagagctg 1740
cgcgtggagg ggtggaaagc ccttcatgag ctgaacccca gggcggccga cgaggccgcg 1800
gtcaactggg tgttcgtgac agacacgctc aacttctcct tttggtcgga gcaggacgag 1860
cacaagtgtg tggtgaggta cagagggaaa acatacagtg ggtactggtc cctgtgcgcc 1920
gccgtcaaca gagccctcga cgaag 1945

<210> 7
<211> 2379
<212> DNA
<213> Homo sapiens

<400> 7
aagcttgtgg tttacttgga cctctgcctc atctttcttc ttttgcgctt cagcctgcgc 60
attcgcttcc tccactaggc tctcatggtg cagaggtttc caagaagatg gtgtgaaggc 120
cgagatcatt tggttatatt ataaaataga atgcaaattc acacaagttt ttgtttttta 180
tttatttatt tttttagaga tgaggtcttg ctatgttgtt tagtctggtc tcgaactcct 240
ggcctcgtga tcctcccacc ttgacctccc aaagtgctgg gattacaggc ctgaggcctg 300
agccactaca cccaactgaa ttcacatttt tttttttctt ttctgagacg gagtctcact 360
ctgtcaccca gtatggagtg cagtggcgcg actgcggctc actgcaagct ccgtctctcg 420
ggttcaagtg attctcatgc ctcagccccc caagtagctg gaattacagg ggtgcactac 480
cacacctggc taatttttct gttttagtag agatggggtt tcaccatgtt gcctggtctc 540
aaactcctga ctttaagtga tccacacacc tcagcctccc aaagtgctgg gattacaggt 600
gtgagcctcc acacccggcc gaattcacat gaattttaaa gtgatgtctt caaagtggtt 660
tcactgtggg gatgggcagc tttttgttat acatctagaa cgttcctctt ctgtttctat 720
gaatactcgg ttggaaaggg ctgaaaaacg gtcttaagag attatctgat tcgtttccca 780
gttttattac tcacatatca gctgtaattt gagcacgttt tctgattgag acaagactca 840
gatggtatta aacattacta caacacatcc gggcacggtg gctcacgcct gtaatcccag 900
cactttggga ggccgaggcg ggcggatcac gaggtcagga gatcgagacc atcctggcta 960
acacggtgaa gccctgtctc tactaaaaat acaaaaaatt aggcgggcat ggtggcgggc 1020
gcctgtagtc ccagctactc gggaggctga ggcaggagaa tggcgtgaac ccgggaggcg 1080
gagcttgcag tgagccgaga tcgcgccact gcactccagc ctgggcgaca gagcaagact 1140
ccatctcaaa aaaaaaaaaa aaaaaaaaaa actacaacac tataaattca tatctattat 1200
aatagtactt tgtgcagggc cctaccctaa gtccttaacc gaacccggaa gcgagaagat 1260
gacttttgtt tgtttttaga gatgggcgcc tggctctgtc gccagcctgg agtgtggggg 1320
cgcgatctcg actcacagca gcctccacct cccgagttca ggcgatcttc ctgcctcagc 1380
ccctcgagga gctgggacca ccggcgcgct ccatcgcgcc cggctaggag ctgactttga 1440
atccgggctc tgcgcctggc cttctgcatc tctataaggg aagacatctg tgacctcggg 1500
gcaaaggtca aattagatcc tgggtaggat cctgttcccg ctgcccctcg ggctggcact 1560
gccaggagta ctcagagctc aaagctggga tctgcagtcc cttacccact cagtgcacgc 1620
cgcctaaggc tttgcgcttc acctttactc acctcgaagc cctggacatc cgcatctgcc 1680
ctaagacttc tcacctcagt agcagaagga agtcgcgtca gctggccaca gcctctctcc 1740
taggagaccg tccgggaaaa gcgagtcagg gtagaccctg aggcccctca gctccggcta 1800

ttttcagatc tgtcgctcct tcaccctcag cctttcaaac aggccactcc aaaaaaaagc 1860
ccaatcacag ccttccttct tctcctggcc ttccggcact gtccaatcaa cgtacgccat 1920
ctatcggtta gtggtgttgc ggggccaccc ttcccgctgg tttccctcgt ggtgtgtaaa 1980
ggcagagagg aaaggcgagg ggtgttgacg ccaggaaggt tccatcttgg ttaagggcag 2040
gagtccctta cggacttgtc tgaggaaaga caggaaagcg ccagcatctc caccttcccc 2100
ggaagcctcc ctttgccagg cagaaagggt ttcccatggg gccgcccctg gcgccgcgcc 2160
cggcccacgt acccggggag gccgggcccc ggaggacgag ggaaagcagg ccgggcgccg 2220
tgagcttcgc ggacgtggcc gtgtacttct ctcccgagga gtgggaatgc ctgcggccag 2280
cgcagagggc cctgtaccgg gacgtgatgc gggagacctt cggccacctg ggcgcgctgg 2340
gtgaggccgg gccctccggc cgggaccccc agtccgtcg 2379

<210> 8
<211> 933
<212> DNA
<213> Homo sapiens

<400> 8
gagacgtact ctggctctgt cgcccaggct ggagcgcaat ggcgccatct cggcgcactg 60
caacctccac ctcccgggtt caagcgattc tactgcctca gcctcccgag tagctgggac 120
tacaggcgcg cactaccaag cccggctaat ttcttttgta tttttagtag agactgggtt 180
tcacgatgtt ggccgggctg gtctggaagt cttgacctca agcgtgcgcc ctctccgcca 240
ctgggtaagg cggggggcgg aatagggggc ttgcaatttc acactagagg cgggcgccgt 300
gggggaaaga agagtcacgt ctcccacggt tcgtagagga aggcctgcct gagcctggag 360
cgggggcggg agagccacag tttggcatcc ccagggcatc ccccagcccg cagactacca 420
ggcctccaga ggacaggacc ccacccccgg ccacaggccc tgcccccagc actccccgca 480
ccccgcctcc aagactcctc cgcccactcc gcacccaact tataaaaacc gtcctcgggc 540
gcggcgggga gaagccgagc tgagcggatc ctcacacgac tgtgatccga ttctttccag 600
cggcttctgc aaccaagcgg gtcttacccc cggtcctccg cgtctccagt cctcgcacct 660
ggaaccccaa cgtccccgag agtccccgaa tccccgctcc caggctacct aagaggatga 720
gcggtgctcc gacggccggg gcagccctga tgctctgcgc cgccaccgcc gtgctactga 780
gcgctcaggg cggacccgtg cagtccaagt cgccgcgctt tgcgtcctgg gacgagatga 840
atgtcctggc gcacggactc ctgcggctcg gccaggggct gcgcgaacac gcggagcgca 900
cccgcagcca gctgagcgcg ctggagcggc gcc 933

<210> 9
<211> 6096
<212> DNA
<213> Homo sapiens

<400> 9
   atctgcacct cctcatatag ggttgatcca agtttcacag acatcactga gttcttagtg 60
   gactcagcta ttggggctgt tctcacactt ttttttcttt tgcaagaatc agcaatgggt 120
   gcaagtggac ctgtgtagga cgtccagtga aacattgtgt tggtgaatca gctagaatcc 180
   atccaagaac tcagccagcc tggtgtgggg tgagatctga tccttgaatg tccctcagtg 240
   gcttttaggg ctggcaggtt cagaagggcc ctctcatcac ccccccaggg cctcattcct 300
   tgtttaacac tttgctatca cagtcttgaa tccttgtaat tgaacaatgg accccacatt 360
   ttcactttgc actggtttct gattctgtaa ccgatcctgt ccccctctct tgtctcattc 420
   actctgggaa ttgtccccac attctgagac ctttcagcag tgccccaacg aggttcctgc 480
   ccttatctga agctccaccc tcacccccat ggcggcaccg caggcagccc tgcttttgcg 540
   tcccgcgtag gcaggctgtg caccggagtc acgaccccct gattcagcct aggcagccac 600
   agcttgactg ctcccgccgg acaagcccta ctgtgctatc tgccgctctt cccttcctct 660
   tcccaggggg tccgcgtcag gggaggcgca gctgtgtgca ttccgggagc ttcagacccc 720
   cgtgtccagc agctccttcg tttcctgggt gctggggcgg ccttcccagc gaagagctca 780
   actcagcggg acgtttggag gctctctgcc ccaaggcgct ggggagtgtg cggcgggaca 840
   gtcgtgcttg cctttttcac tttcagagtg tccacgcccc acccgtttgg tcactgcagg 900
   tcagtccagt ccagcccggc ccaccccacc ggtgcgtgtc tgtcgcacgt ggcagacgcc 960
   atactctctg ttcttgttta aagcccagga tctactgggc cctggaggca agaggtgaac 1020
   gcagcggaat ccacgctgag ctgcccggga acggagcttc caaccccaga aggaggactc 1080
   tgtgctccta caccttaacc ctttttagcc cgaaacttct ccaacttcct tggctttgtt 1140
   tagagctcga cagcgccgcc ccctggcgct cgttgtgagg acagtagagg agagaggcaa 1200
   gggtgttttt aaacagtttg cctctcacca ttatgggggc gacccgaggg ggagacccac 1260
   tcttccgcat tcccggtaag tgaaccaccg gaagaggtcg aaagtgacgg attcccatgt 1320
   cctcctccag cccccccccc accctgccca tccacaggac ggtggctctt cagtgccctt 1380
   tgccgagcaa gtggcgtttc tatgcacgtg ggtatcaatt cggactctgg acgaaatgga 1440
   aacctcctta gccgacccgg gtgggatcag ctgggatcct gcgcgctccc ctggggggtt 1500
   gccagccact ctgttggggt gcaagaagca ccatccttcg gaagctgggc cgaaactggc 1560
   caggctgact cgctcccacg cgcccgcccc tacccggcgc cgcagcaatt cacctgccac 1620
   cgcctctgag ccgggtccgg acttcggcgc cctgacagtg tccccgcgac ttccccaccc 1680
   gatgagatgg ggtctggcgt tggccagtgc gtgtccaggg actcgcgggt ccctggccag 1740
   ccatggggca gagggcgctg gtgttaggcc agtcttcccc accctgcccc gtcaccccag 1800
   ccacacccac tgtcctgtga ggccaagcgc gctccgctgg tttcctgagc caggcacctt 1860
   ggccgcggac aggatccagc tgtctctcct tgcgatcctg tcttcgggga agtccacgtc 1920
   ctaggcaggt cctcccaaag tgcccttggt gccgatcacc cctcccagcg tcttgcaggt 1980
   cctgtgcacc acctccccca ctccccattc aaagccctct tctctgaagt ctccggttcc 2040
   cagagctctt gcaatccagg ctttccttgg aagtggctgt aacatgtatg aaaagaaaga 2100
   aaggaggacc aagagatgaa agagggctgc acgcgtgggg gcccgagtgg tgggcgggga 2160
   cagtcgtctt gttacagggg tgctggcctt ccctggcgcc tgcccctgtc ggccccgccc 2220
   gagaacctcc ctgcgccagg gcagggttta ctcatcccgg cgaggtgatc ccatgcgcga 2280
   gggcgggcgc aagggcggcc agagaaccca gcaatccgag tatgcggcat cagcccttcc 2340
   caccaggcac ttccttcctt ttcccgaacg tccagggagg gagggccggg cacttataaa 2400
   ctcgagccct ggccgatccg catgtcagag gctgcctcgc aggggctgcg cgcagcggca 2460
   agaagtgtct gggctgggac ggacaggaga ggctgtcgcc atcggcgtcc tgtgcccctc 2520
   tgctccggca cggccctgtc gcagtgcccg cgctttcccc ggcgcctgca cgcggcgcgc 2580
   ctgggtaaca tgcttggggt cctggtcctt ggcgcgctgg ccctggccgg cctggggttc 2640
   cccgcacccg cagagccgca gccgggtggc agccagtgcg tcgagcacga ctgcttcgcg 2700
   ctctacccgg gccccgcgac cttcctcaat gccagtcaga tctgcgacgg actgcggggc 2760
   cacctaatga cagtgcgctc ctcggtggct gccgatgtca tttccttgct actgaacggc 2820
   gacggcggcg ttggccgccg gcgcctctgg atcggcctgc agctgccacc cggctgcggc 2880
   gaccccaagc gcctcgggcc cctgcgcggc ttccagtggg ttacgggaga caacaacacc 2940
   agctatagca ggtgggcacg gctcgacctc aatggggctc ccctctgcgg cccgttgtgc 3000
   gtcgctgtct ccgctgctga ggccactgtg cccagcgagc cgatctggga ggagcagcag 3060
   tgcgaagtga aggccgatgg cttcctctgc gagttccact tcccagccac ctgcaggcca 3120
   ctggctgtgg agcccggcgc cgcggctgcc gccgtctcga tcacctacgg caccccgttc 3180
   gcggcccgcg gagcggactt ccaggcgctg ccggtgggca gctccgccgc ggtggctccc 3240
   ctcggcttac agctaatgtg caccgcgccg cccggagcgg tccaggggca ctgggccagg 3300
   gaggcgccgg gcgcttggga ctgcagcgtg gagaacggcg gctgcgagca cgcgtgcaat 3360
   gcgatccctg gggctccccg ctgccagtgc ccagccggcg ccgccctgca ggcagacggg 3420
   cgctcctgca ccgcatccgc gacgcagtcc tgcaacgacc tctgcgagca cttctgcgtt 3480
   cccaaccccg accagccggg ctcctactcg tgcatgtgcg agaccggcta ccggctggcg 3540
   gccgaccaac accggtgcga ggacgtggat gactgcatac tggagcccag tccgtgtccg 3600
   cagcgctgtg tcaacacaca gggtggcttc gagtgccact gctaccctaa ctacgacctg 3660
   gtggacggcg agtgtgtgga gcccgtggac ccgtgcttca gagccaactg cgagtaccag 3720
   tgccagcccc tgaaccaaac tagctacctc tgcgtctgcg ccgagggctt cgcgcccatt 3780
   ccccacgagc cgcacaggtg ccagatgttt tgcaaccaga ctgcctgtcc agccgactgc 3840
   gaccccaaca cccaggctag ctgtgagtgc cctgaaggct acatcctgga cgacggtttc 3900
   atctgcacgg acatcgacga gtgcgaaaac ggcggcttct gctccggggt gtgccacaac 3960
   ctccccggta ccttcgagtg catctgcggg cccgactcgg cccttgcccg ccacattggc 4020
   accgactgtg actccggcaa ggtggacggt ggcgacagcg gctctggcga gcccccgccc 4080
   agcccgacgc ccggctccac cttgactcct ccggccgtgg ggctcgtgca ttcgggcttg 4140
   ctcataggca tctccatcgc gagcctgtgc ctggtggtgg cgcttttggc gctcctctgc 4200
   cacctgcgca agaagcaggg cgccgccagg gccaagatgg agtacaagtg cgcggcccct 4260
   tccaaggagg tagtgctgca gcacgtgcgg accgagcgga cgccgcagag actctgagcg 4320
   gcctccgtcc aggagcctgg ctccgtccag gagcctgtgc ctcctcaccc ccagctttgc 4380
   taccaaagca ccttagctgg cattacagct ggagaagacc ctccccgcac cccccaagct 4440
   gttttcttct attccatggc taactggcga gggggtgatt agagggagga gaatgagcct 4500
   cggcctcttc cgtgacgtca ctggaccact gggcaatgat ggcaattttg taacgaagac 4560
   acagactgcg atttgtccca ggtcctcact accgggcgca ggagggtgag cgttattggt 4620
   cggcagcctt ctgggcagac cttgacctcg tgggctaggg atgactaaaa tatttatttt 4680
   ttttaagtat ttaggttttt gtttgtttcc tttgttctta cctgtatgtc tccagtatcc 4740
   actttgcaca gctctccggt ctctctctct ctacaaactc ccacttgtca tgtgacaggt 4800
   aaactatctt ggtgaatttt tttttcctag ccctctcaca tttatgaagc aagccccact 4860
   tattccccat tcttcctagt tttctcctcc caggaactgg gccaactcac ctgagtcacc 4920
   ctacctgtgc ctgaccctac ttcttttgct cttagctgtc tgctcagaca gaacccctac 4980
   atgaaacaga aacaaaaaca ctaaaaataa aaatggccat ttgctttttc accagatttg 5040
   ctaatttatc ctgaaatttc agattcccag agcaaaataa ttttaaacaa aggttgagat 5100
   gtaaaaggta ttaaattgat gttgctggac tgtcatagaa attacaccca aagaggtatt 5160
   tatctttact tttaaacagt gagcctgaat tttgttgctg ttttgatttg tactgaaaaa 5220
   tggtaattgt tgctaatctt cttatgcaat ttcctttttt gttattatta cttatttttg 5280
   acagtgttga aaatgttcag aaggttgctc tagattgaga gaagagacaa acacctccca 5340
   ggagacagtt caagaaagct tcaaactgca tgattcatgc caattagcaa ttgactgtca 5400
   ctgttccttg tcactggtag accaaaataa aaccagctct actggtcttg tggaattggg 5460
   agcttgggaa tggatcctgg aggatgccca attagggcct agccttaatc aggtcctcag 5520
   agaatttcta ccatttcaga gaggcctttt ggaatgtggc ccctgaacaa gaattggaag 5580
   ctgccctgcc catgggagct ggttagaaat gcagaatcct aggctccacc ccatccagtt 5640
   catgagaatc tatatttaac aagatctgca gggggtgtgt ctgctcagta atttgaggac 5700
   aaccattcca gactgcttcc aattttctgg aatacatgaa atatagatca gttataagta 5760
   gcaggccaag tcaggccctt attttcaaga aactgaggaa ttttctttgt gtagctttgc 5820
   tctttggtag aaaaggctag gtacacagct ctagacactg ccacacaggg tctgcaaggt 5880
   ctttggttca gctaagctag gaatgaaatc ctgcttcagt gtatggaaat aaatgtatca 5940
   tagaaatgta acttttgtaa gacaaaggtt ttcctcttct attttgtaaa ctcaaaatat 6000
   ttgtacatag ttatttattt attggagata atctagaaca caggcaaaat ccttgcttat 6060
   gacatcactt gtacaaaata aacaaataac aatgtg 6096

<210> 10
<211> 2500
<212> DNA
<213> Homo sapiens

<400> 10
acccacttct gtgtgtggat agtatcctgc aggagagatg ttgtctgcag tgtgagctgg 60
gcccaccgga gtgtgtgaat aggatcctgc aggagaaatg gaatccggag tgtgagctgc 120
atccgctgta gagggtggat aaaatcctgc aggaaagatg gcatctggaa tgtcagcggg 180
agccaccgac ctctgaggat gcaccccgca ggtgtgatgc ggggccagtt ccaaggctgg 240
gttaggtttt accctggctt ctgtgttgta ctctcattct cttcctcttt cttctaatac 300
ctgctctggg aggcatcagg ccatgtccag tgtgcaggcc atggagaccc acacggcaag 360
gaactggaac cccctgccag cagcctcggg ggtccagtcc ttagatggtg ccctgtggtc 420
agcaatgcac ctgtgacctc cgggctatgt ctcgtggtag ttgcttttgt gttttaacat 480
agcaacagga aactagccta ttacccacca atcccattcc aggctgcttt caaacgcagc 540
tcaggctaga acaccagcac ggggacacag ctgagacttg gggtttgcga cgggaacacg 600
cccatgctgt gcctctgaat ctggcaccgt caccctgtgg cctgggttca gcaacttggc 660
ctcaccttcc ttgtctgtga aattcagact gggtccttgt gagatgattg gagagaatgt 720
atgaactatg tgagaacgcc acctttgtgc gtatctcacg cagtgtcttc cctcctttcc 780
aaagtcttct gctgtctcta gacacacccg acgtgggggg ggggggttcc ctgggtctcc 840
tcctaggtct gtcccaggag ggcacgcact gaaggccgcg agaatcccgg gggctgcatt 900
gcgccgcgcc aaggactcca cacaggacct ttcattttcc caactgtgct gagccaggcg 960
gccggcagag agcaggtggc tgacaggccc cggggagccg gaccgcctgg gtctaatctt 1020
cccgcagact cccttgctgt gcgctttggg gcttgggcct cagtttcctc aaaaggaatg 1080
aggggctttt ttggaacgtt aaataatttc ctacgtggtt gcgggtaggg agaaggagaa 1140
agagaggagc gcgcctgcgc gcctggaatc gtgcccggat cagagcaagc gctctaaaag 1200
tgttacaaac attaaggcgc caactaaaaa acccgtagtg agcgcaggca gaaaccacgg 1260
gtaagagaag tggagaagct tcgcgtaggc cccagggtcc cgagccccga gtctcgagcg 1320
cagaatcagg ggtgccaatg ctctcctccg cgcccccgag cgctcgcctt ggccatgcgg 1380
gccgccccac cgggatgagg gcgctcaggc cggacgctgg ggccccgggt tctcgccccg 1440
ccccgccctc ggggattcag aggggccggg aggagcctcg cgcatgtgca cagctggcgc 1500
cccccgcccc ccgcgcacag ctgggacgtg ggccgcggcc gggcgggcgc agtcgggagc 1560
cggccgtggt ggctccgtgc gtccgagcgt ccgtccgcgc cgtcggccat ggccaagcgc 1620
tccaggggcc ccgggcgccg ctgcctgttg gcgctcgtgc tgttctgcgc ctgggggacg 1680
ctggccgtgg tggcccagaa gccgggcgca gggtgtccga gccgctgcct gtgcttccgc 1740
accaccgtgc gctgcatgca tctgctgctg gaggccgtgc ccgccgtggc gccgcagacc 1800
tccatcctgt gagtgccgcg ggggacgccg ggggcgcggg gtccggggct tcgtggagat 1860
ccgggagcgc aggggtgatc ggaggtgggg ggcgcggagg gtggaggggg catcgggcgc 1920
gcggggggcc tggggacttg ggacgcagaa gggaacctcc gaagggggac gtggggggac 1980
ctgggcgcgg ggacccgctg ggcctttgtt cgccctgcgg gagacgccga ggggcggaac 2040
agagcgctgt gcgcgcggcc ttcgtagccg cctttgttcg gaactcggaa tccccgcagg 2100
actgggaagt tgttggagcc tccggggctc cccccgctcg cctcccgccg ccccctctca 2160
tgctccgccg gcctcccgct tccccctggt tcgcggcccc tcctccgctc acctttcccc 2220
cgctcaggac ccctcggtcc ccctccgctc cccgagcgcg gcgcagcccc ctccgtcctc 2280
ccagccccct ccgccccgtt cctcgtcctg ttcgctcccc tcctccgctc ctcttcctcc 2340
tccccttcct cctcctcctc cccttcctcc tcctcctccc cttcctcctc ctcctccctt 2400
cccctcctcc tccccccctt ccttctcctc ccccagcctc cgccctctcc ccctcccccg 2460
ccccttggag cgcagtgccc accccatccc cccgcgccgg 2500

<210> 11
<211> 2200
<212> DNA
<213> Homo sapiens

<400> 11
cctcgccccc tccagccggc cccccgggcc cctcctctcg gcgcccggac cttggccctc 60
cctctccttt cccacttctc tctttgccct aacttcgccc ccatcccccg ctcatttcct 120
ctcgcacccg ggctcgccaa tccctctttc caagtccctc ttccagcccg gccttcctct 180
cgggttcgcc ccccttctcc ccaatctccg tcctcttccc tcccttcgcc ctccccccct 240
tccttcctct tcccctcacc caaccctggt tcccctcgtt cctcagtccc gatctctccc 300
ttactctgtc cccgcccact ctgcgccggc ctctcagtcc gggttgagcc ccacgtgtgg 360

acggccgcgc ccccactgac agccgccgcc cgccggcccg ccccgcgccc cgccgggcct 420
ctaaaacccc cgcgccgcgc cctccaccgc cgcatcttct ccagcgccca gcctcccgcc 480
ctctctcttg ctggccgcac gccccggccc cgcgcacctc cgcccggctc cgcagccgct 540
acccgcgctt cgttgccctg tgggactccg agcgagcccg gagggaaccc tcctcttctt 600
ctgggggcga cttttgtttg cttgcctgtt tctttctggt gacttttgca gctttccaat 660
atccgtcttc ggagcgcacg ggaatccgcc gagctctgcg tgcaggccct tttttctttt 720
gaggttcaca ttttttgaaa ttttacgcca gggcttttgt aatttcctcc cccgcccgct 780
gacggtcctg gagtcgctcg gggctttagg ccggttatgc aacgtgtacc gctcggggct 840
gccggctgca cctccgccgc gcctcgccgc tcactgcgct agacccggcg ccccgcgtct 900
cgcttcgcgg gcagtcaggg ggccggcgct ctgtcgaggt ctccagctag agcagggagc 960
ccgagcccga gggagtcccc ggagccgacg aagggcttat tagaccctga ctcttttctg 1020
aggcgcgcag attttgtctt tgatcactcc ctctccgcgg gtctacggcc gcgcgctttc 1080
ggcgccggcg atggggagaa gacggaggct gtgtctccag ctctacttcc tgtggctggg 1140
ctgtgtggtg ctctgggcgc agggcacggc cggccagcct cagcctcctc cgcccaagcc 1200
gccccggccc cagccgccgc cgcaacaggt tcggtccgct acagcaggct ctgaaggcgg 1260
gtttctagcg cccgagtatc gcgaggaggg tgccgcagtg gccagccgcg tccgccggcg 1320
aggacagcag gacgtgctcc gagggtaagt gggcaagcgg ctccgcacct agggctccgg 1380
cttgggggag gggggaatcc tcagtttggc ggctttctgg cccactccgt cccagaccct 1440
ttagctggag cctagagctg cagccccctt tgccagaata tccaaagacc cccaggagcg 1500
cgtccccctt ttccttccca accccgcagc tcagcgggcg gaaagccctc tctccggggg 1560
ttgggcggcg ggtggttagg gggtccaggg gtgccgatcg cagagcgtgt gcagagctcg 1620
cgctgcggga acaggttctg aatgtccggc ggcaggcggg cctgggtccg cctgctgcag 1680
gggccagaga agcctgcttg ctccccacgt cggggccgcc gctcgtgagc cttttgtttg 1740
aggacgtgtg cagggttcac agctcacctt ctcatcgtca acccgagcgc tccaccttgc 1800
gacgcgcttt ccttgacacg tcggggccaa agtaacagtt gaccaaggag gaatggattt 1860
gggaaggagg gcaaggattc tttggaacgg aatggtccct ttgttctctg catctggaag 1920
ctagaatagt agcaaattat atgtttccat gcctcttttc gccctttaaa aggcaggcaa 1980
agggacgaca gatgaaaggc agtgtttaga catttctgac cctcctgcat tccagcatct 2040
agctcttttg cttccacgtc tgcctcccga tctccaataa tttgaagtgt aattttgatt 2100
tgtttgttgt cctgaaatct actcgctcgg ggcattgctt acgaagaccg tttatatgtt 2160
gctgcatccc tctacctatc tgttacgtga ccgcgcttgt 2200

<210> 12
<211> 2000
<212> DNA
<213> Homo sapiens

<400> 12
ttggaagaaa aggatctccg aggaaggggc tgagagaagg gcagggtgaa ctggactaaa 60
ggccagagta ggaaggagaa gaggggccaa aaaagaaggg gatgaaatta agcacagaag 120
atgggtaaag aaaaaagtat cagggaaagg gcaaaataag agaaagcctt gaggataaga 180
gggtagaagg ctaaagaaca aggggaccac tgggtcgggg aagcgctgcc tgaacggcgg 240
gacagtgaca aagaaagggc gctggcgata ttcgcaccaa gggtgcgaaa cgcaatcggg 300
aggtgagaaa tggaaagaag gcgaatgccc ggctacaagt agcctgggac tgaaagggga 360
cctgggggag gggctgggcc cagggcagaa aagtccaggt tcccatgcgg cctgggccca 420
cgtggagcgg gcgctgaatc accgttcagc cgcccccctc ccctcctccc cgaccggtgc 480
ccgcagtccc cgcctcctcg gccgccgcct ccacggggcg gggccctggc ccgggaccag 540
cgccgcggct ataaatgggc tgcggcgagg ccggcagaac gctgtgacag ccacacgccc 600
caaggcctcc aagatgagct acacgttgga ctcgctgggc aacccgtccg cctaccggcg 660
ggtaaccgag acccgctcga gcttcagccg cgtcagcggc tccccgtcca gtggcttccg 720
ctcgcagtcg tggtcccgcg gctcgcccag caccgtgtcc tcctcctata agcgcagcat 780
gctcgccccg cgcctcgctt acagctcggc catgctcagc tccgccgaga gcagccttga 840
cttcagccag tcctcgtccc tgctcaacgg cggctccgga cccggcggcg actacaagct 900
gtcccgctcc aacgagaagg agcagctgca ggggctgaac gaccgctttg ccggctacat 960
agagaaggtg cactacctgg agcagcagaa taaggagatt gaggcggaga tccaggcgct 1020
gcggcagaag caggcctcgc acgcccagct gggcgacgcg tacgaccagg agatccgcga 1080
gctgcgcgcc accctggaga tggtgaacca cgagaaggct caggtgcagc tggactcgga 1140
ccacctggag gaagacatcc accggctcaa ggagcgcttt gaggaggagg cgcggttgcg 1200
cgacgacact gaggcggcca tccgcgcgct gcgcaaagac atcgaggagg cgtcgctggt 1260
caaggtggag ctggacaaga aggtgcagtc gctgcaggat gaggtggcct tcctgcggag 1320
caaccacgag gaggaggtgg ccgaccttct ggcccagatc caggcatcgc acatcacggt 1380
ggagcgcaaa gactacctga agacagacat ctcgacggcg ctgaaggaaa tccgctccca 1440
gctcgaaagc cactcagacc agaatatgca ccaggccgaa gagtggttca aatgccgcta 1500
cgccaagctc accgaggcgg ccgagcagaa caaggaggcc atccgctccg ccaaggaaga 1560
gatcgccgag taccggcgcc agctgcagtc caagagcatc gagctagagt cggtgcgcgg 1620
caccaaggag tccctggagc ggcagctcag cgacatcgag gagcgccaca accacgacct 1680
cagcagctac caggtaggaa ccgcggctgc gcggccagcc tgcgccagcg ccagcgccgc 1740
gcgcccccga cacttgggct cgtgcccagg cgccctctcc gccgcgctcc ctggtggccg 1800

ctcgctagag cacgcgcgcc gcagacctag ggtatttgcg gatcagcgtc ctcgcccatc 1860
tcatcctcca cactccgccc ccacccacct gccccagctg ctaagggtct tgaccttttt 1920
cagaaacgtg catcttttcc agttctaatt ttgcacgctt gcacgtttaa agcaggaggg 1980
atgaattcgg tagtggataa 2000

<210> 13
<211> 2300
<212> DNA
<213> Homo sapiens

<400> 13
tcagattgtc attgggaggg tgaataaatg aatgcttgca ttatgagagt ttgggggcag 60
aaatatgcca cagactctta tctgaagcca tcagatttag tggctgcgaa cccaccgaag 120
tcagggattt acatttttta cagcaacgag agaaaacttc ccctttcctc tgcagaagtc 180
aggactggat ctcaaaaata gaaatgtgtc ctcctaaatg tgtgcccatc cccgtggttg 240
acaaacaacg gatttcccaa gatagctgcc acacacttgg tttctaatct ctgtattgct 300
tccccgccag aatgtcgaag tccttcccga atatgcccag tcatactttc tgaacttttg 360
agcaaacacc gtccggcttc ttgtgctttc ctcaaagacc ccaggcaccg gcagggagga 420
cacaggccgg ggcagagcgc ccctgcgcgg gggattcctg ccactccgcg ccagcctgcg 480
gcgcaaacgc tcttctcagc cgcagtccca cccgctgctg gcaatctgaa tgaggagccg 540
cgctattttt acctccccgg ctgcaatcct ttatatttac atgcaggaag caaatatata 600
agggattaag aaggagatgc gtggccttag tttatccaga gcaggaagag gttggaatag 660
gagagggtat gtgaagtctg ggtggtggaa aaaggcaggt ggacttcggc tggttgtttt 720
ctcccgatca tccctgtctc tggcctggaa acccccgtac tctctttctt ctggcttatc 780
cgtgactgcc ggctccccct ccaccgcccc catcttttga ggtaccaccc gtcacctccg 840
atgctgcttg ggctgctgca tcactctgct gctttacccc cttccccgcc ccccaacaaa 900
gcatgcgcag tgcgttccgg gccaggcaac agcagcagca cagcatccag caacagcatc 960
agcacccgaa gccccgctcg ggcgcgctct cggggggcgg ggcgcacgcc cgctccgcgc 1020
gtccccgcgc cgctcgctcc cgcgcgtccc cgcgccgctc gctcccgcgc gccgcctcag 1080
catcctcagg cccggcggca gcccccgcag tcgctgaagc ggccgcgccc gccgggggag 1140
ggagtagccg ctggggaggc tccaagttgg cggagcggcg aggacccctg gactcctctg 1200
cgtcccgccc cgggagtggc tgcgaggcta ggcgagccgg gaaagggggc gccgcccagc 1260
cccgagcccc gcgccccgtg ccccgagccc ggagccccct gcccgccgcg gcaccatgcg 1320
cgccgagccg gcgtgaccgg ctccgcccgc ggccgccccg cagctagccc ggcgctctcg 1380
ccggccacac ggagcggcgc ccgggagcta tgagccatga agccgcccgg cagcagctcg 1440
cggcagccgc ccctggcggg ctgcagcctt gccggcgctt cctgcggccc ccaacgcggc 1500
cccgccggct cggtgcctgc cagcgccccg gcccgcacgc cgccctgccg cctgcttctc 1560
gtccttctcc tgctgcctcc gctcgccgcc tcgtcccggc cccgcgcctg gggggctgct 1620
gcgcccagcg gtgggtatgg ccccgtgccc tttgcgttgg ctttcccgcg gggccctgca 1680
gaggaaagcg aagggcgcgc gggtccgtgt gctccgggct tgtccccggc tcggcctttc 1740
cttccctccc tgcctgtctt tccacccttc tcgttcccaa acccccattc atcccagttc 1800
acttttggaa gtccatttct gttgcattcg cgaaaaaccc attccaattc ttgttggttc 1860
cactgggagg tgtttagtgg atcctgggtc cctcagcgat ctctgtgcaa cttgcggagg 1920
ggcaaccagt ggatgggaaa tacagcgagg gagcaagttg ctacttgcgt ggtggaacct 1980
taatgtgaat gcggggagga tgtagtgata atagtggtaa tgggctgttt cctcaaattt 2040
cgtatccggc gcattcagtg cggttggaat taaggtgggg gaggcacact tcggggacca 2100
aagaattaag gtgctgaaga catacttcat gcacgacctt tggttctgat ttctcaaagt 2160
gcttgtcatt ataatgaaca attaatataa taccatcttc tatatattga tgattggaag 2220
tcactgaaag cagaaagctg gctttgtcag gaaaataaaa agaaattggg aagctgccag 2280
catctgtatc cctacatggc 2300

<210> 14
<211> 3000
<212> DNA
<213> Homo sapiens

<400> 14
tactgccgac tttaggtctc tctggatctc aggccccctt ctctaagatg catcctagag 60
gaccaaaaat acactttatt tgggcttcgc ctgcttttgt ggaagggtag tttactagag 120
gatataatct cgtgttttaa tttgctctct ctcctaaagg aaatgtggag aaaaaaaaaa 180
agcagaaatt ggaaataacc aatatttagt ttatttcatt cgattcttag gggaactggt 240
gaggagccta agatgatttt cccttcctag agaaagaatc caaagtccag ggaaatagcg 300
acaggggagt tcaagactgc ccctgctagt ccttccttgg ctactctccg ctgcgatcgc 360
aggatagctc tcattagcag gagaatcggg caagtgtgtg gataagtaga gagtgtgttg 420
aacaacttgt aacgttttat gaaatacgca ttgtcatggt tccctaaaag gctttgcgga 480
agccgtttgt ctttactaat caagtcttta cttacacaaa agtagaagta gaagtagttt 540
tagaaaacat actaacaatc ttctatcccc ttgaagacca gagtagcaga aaacaggtga 600
tttgcattat aaaattgcac tcactttttc ctcctttcag atttcacatt acattagccc 660

atttgtgtta cggtgtataa aaaatggaac aggcgcctcc actgcattgt tctcctttaa 720
aaatagatca cttacaccct aactttgttt tccttaaatt cgattcttaa caggagagct 780
ttctattatt tcagatggag tgaggttgca cgactgggat ggaagaaagg aatcccttaa 840
atttggggga atttctgttc tctgttccaa gaccatttta cttggggtgt gggggtgggc 900
gcggcggtca gggcagtgga acgcagtcgc ggctgcgcca tccctgcact tccaggcgcg 960
cgggagggac cggcggggac gcgagctgcg gactctggcg aactcggggg aggcagacag 1020
ggggaggcgg acacccagcc ggcaggcgtc tcagcctccc cgcagccggc gggcttttct 1080
cctgacagct ccaggaaagg cagacccctt ccccagccag ccaggtaagg taaagactgc 1140
tgttgagctt gctgttactg agggcgcaca gaccctgggg agaccgaagc ttgccactgc 1200
gggattctgt ggggtaacct gggtctacgg aagtttcctg aaagagggga gaagggtttg 1260
catttttcct atggaggatt cttctctctc tagcatttcg tttgatgtat tcaactggta 1320
gaagtgagat ttcaacaggt agcagagagc gctcacgtgg aggaggtttg gggcgccgcg 1380
gcgccacccc cacccctcct cgggaccgcg cctatttcta aagttacacg tcgacgaact 1440
aacctatgct ttaaattcct ctttccagcc ccgtgagtcc gcggcgacat tgggccgtgg 1500
ggtggctggg aacggtcccc tcctccggaa aaaccagaga acggcttgga gagctgaaac 1560
gagcgtccgc gagcaggtcc gtgcagaacc gggcttcagg accgctgagc tccgtagggc 1620
gtccttgggg gacgccaggt cgccggctcc tctgccctcg ttgagatgga caacgcctcg 1680
ttctcggagc cctggcccgc caacgcatcg ggcccggacc cggcgctgag ctgctccaac 1740
gcgtcgactc tggcgccgct gccggcgccg ctggcggtgg ctgtaccagt tgtctacgcg 1800
gtgatctgcg ccgtgggtct ggcgggcaac tccgccgtgc tgtacgtgtt gctgcgggcg 1860
ccccgcatga agaccgtcac caacctgttc atcctcaacc tggccatcgc cgacgagctc 1920
ttcacgctgg tgctgcccat caacatcgcc gacttcctgc tgcggcagtg gcccttcggg 1980
gagctcatgt gcaagctcat cgtggctatc gaccagtaca acaccttctc cagcctctac 2040
ttcctcaccg tcatgagcgc cgaccgctac ctggtggtgt tggccactgc ggagtcgcgc 2100
cgggtggccg gccgcaccta cagcgccgcg cgcgcggtga gcctggccgt gtgggggatc 2160
gtcacactcg tcgtgctgcc cttcgcagtc ttcgcccggc tagacgacga gcagggccgg 2220
cgccagtgcg tgctagtctt tccgcagccc gaggccttct ggtggcgcgc gagccgcctc 2280
tacacgctcg tgctgggctt cgccatcccc gtgtccacca tctgtgtcct ctataccacc 2340
ctgctgtgcc ggctgcatgc catgcggctg gacagccacg ccaaggccct ggagcgcgcc 2400
aagaagcggg tgaccttcct ggtggtggca atcctggcgg tgtgcctcct ctgctggacg 2460
ccctaccacc tgagcaccgt ggtggcgctc accaccgacc tcccgcagac gccgctggtc 2520
atcgctatct cctacttcat caccagcctg agctacgcca acagctgcct caaccccttc 2580
ctctacgcct tcctggacgc cagcttccgc aggaacctcc gccagctgat aacttgccgc 2640
gcggcagcct gactccccca gcgtccggct ccgcaactgc ccgccactcc tggccagcga 2700
gggaggagcc ggcgccagag tgcgggacca gacaggccgc ctaggcctcc tggggaaacc 2760
gactcgcgcc ccatacccga cctagcagat cggaagcgct gcgactgtgc ccgcaggttg 2820
accttgccaa gccctccagg tgatgcgcgg ccatgccggg tgaggagaac tgaggctgag 2880
atcgccacac tgagggctcc ctaaagccga ggtggaggaa gaggagggta gaggaggagg 2940
gcggtattgc tgggaaccgc cccctccctg ccctgctccc tgctgcccca cccgagccct 3000

<210> 15
<211> 3000
<212> DNA
<213> Homo sapiens

<400> 15
   gaatacatta aagtaggggc aacccttgag cccagacttc tgccatgtga agaccctttg 60
   aaaatcctga caaacacagg tactgcgtaa gtggtcagct aattaaagag gggaggtgga 120
   gctgtccttt gtgtatccaa taagtaccca ttatctcatt tgagcatgaa aagaggccac 180
   tgttattact ttcaagaagg aaagtaagca ggatagctca tatttttaga accattcctc 240
   accaaatgga ataattccgg tgaaaagtgg gagtgaggaa gaaagaaaaa aaaaacttct 300
   aatcataatg tttgggaata agaaaggaag aagaaactca cgtcaaagcc gactttctcc 360
   tgcagctgta aaataaactc ttaagaccct tcctgctgaa actctggaga ggaaaactgg 420
   agtggcgggt gggctttgcc tgcagctcaa ctctccctcg cggcgcgggc gcggctgggt 480
   tcagcacctc ggaaagcgcc cctcgcggcg ccccgggatt acgcatgctc cttggggccc 540
   gccgccttgg ccgtgcaagt gccaccgtaa ctggtgagag ccgctggcaa cccacccgga 600
   gttgacaacc gcggagagac gcagacaccc actgacctcc aggaagctga gcgtggtgga 660
   tggaactcta cgatctcttt ctctccaagg acggaaacct catccaagca gtcccagagg 720
   aaacggataa aggtatttga aagggagcga gcggccccaa atcgcacaat tgagcggctg 780
   ggggagttat gcgccagtgc cccagtgacc gcgggacacg gagaggggaa gtctgcgttg 840
   tacataagga cctagggact ccgagcttgg cctgagaacc cttggacgcc gagtgcttgc 900
   cttacgggct gcactcctca actctgctcc aaagcagccg ctgagctcaa ctcctgcgtc 960
   cagggcgttc gctgcgcgcc aggacgcgct tagtacccag ttcctgggct ctctcttcag 1020
   tagctgcttt gaaagctccc acgcacgtcc cgcaggctag cctggcaaca aaactggggt 1080
   aaaccgtgtt atcttaggtc ttgtccccca gaacatgacc tagaggtacc tgcgcatgca 1140
   gatggccgat gcagccacga tagccaccat gaataaggca gcaggcgggg acaagctagc 1200
   agaactcttc agtctggtcc cggaccttct ggaggcggcc aacacgagtg gtaacgcgtc 1260
   gctgcagctt ccggacttgt ggtgggagct ggggctggag ttgccggacg gcgcgccgcc 1320
   aggacatccc ccgggcagcg gcggggcaga gagcgcggac acagaggccc gggtgcggat 1380
   tctcatcagc gtggtgtact gggtggtgtg cgccctgggg ttggcgggca acctgctggt 1440
   tctctacctg atgaagagca tgcagggctg gcgcaagtcc tctatcaacc tcttcgtcac 1500
   caacctggcg ctgacggact ttcagtttgt gctcaccctg cccttctggg cggtggagaa 1560
   cgctcttgac ttcaaatggc ccttcggcaa ggccatgtgt aagatcgtgt ccatggtgac 1620
   gtccatgaac atgtacgcca gcgtgttctt cctcactgcc atgagtgtga cgcgctacca 1680
   ttcggtggcc tcggctctga agagccaccg gacccgagga cacggccggg gcgactgctg 1740
   cggccggagc ctgggggaca gctgctgctt ctcggccaag gcgctgtgtg tgtggatctg 1800
   ggctttggcc gcgctggcct cgctgcccag tgccattttc tccaccacgg tcaaggtgat 1860
   gggcgaggag ctgtgcctgg tgcgtttccc ggacaagttg ctgggccgcg acaggcagtt 1920
   ctggctgggc ctctaccact cgcagaaggt gctgctgggc ttcgtgctgc cgctgggcat 1980
   cattatcttg tgctacctgc tgctggtgcg cttcatcgcc gaccgccgcg cggcggggac 2040
   caaaggaggg gccgcggtag ccggaggacg cccgaccgga gccagcgccc ggagactgtc 2100
   gaaggtcacc aaatcagtga ccatcgttgt cctgtccttc ttcctgtgtt ggctgcccaa 2160
   ccaggcgctc accacctgga gcatcctcat caagttcaac gcggtgccct tcagccagga 2220
   gtatttcctg tgccaggtat acgcgttccc tgtgagcgtg tgcctagcgc actccaacag 2280
   ctgcctcaac cccgtcctct actgcctcgt gcgccgcgag ttccgcaagg cgctcaagag 2340
   cctgctgtgg cgcatcgcgt ctccttcgat caccagcatg cgccccttca ccgccactac 2400
   caagccggag cacgaggatc aggggctgca ggccccggcg ccgccccacg cggccgcgga 2460
   gccggacctg ctctactacc cacctggcgt cgtggtctac agcggggggc gctacgacct 2520
   gctgcccagc agctctgcct actgacgcag gcctcaggcc cagggcgcgc cgtcggggca 2580
   aggtggcctt ccccgggcgg taaagaggtg aaaggatgaa ggagggctgg ggggggcccc 2640
   atttaagaag taggtgggag gaggatggcc agagcatgga ggaggagcct gtggataggc 2700
   cgaggacctt ctctggagag gagatgcttc gaaatcaggt ggagagagga aattggcaaa 2760
   gggatagaga cgagccccac gggccagaca gccaacctcc gctccgcacc ccacagcctc 2820
   tccttactct tcccacgctg agtagtgtgg gggcgcccag aagcgaagac aagcagcaaa 2880
   aatgtagaga aattggcacg gggagcgggg cttagccaaa tgatgcacag acaattgtgc 2940
   ccgtttattc cagcgacttc tgcggagagg gcagccgtcg gcacaaacac tcctttgcgt 3000

<210> 16
<211> 2200
<212> DNA
<213> Homo sapiens

<400> 16
gtcccccgat tccctcaccc atcatataac gtgtgtattt attatgtttc ccgtttcctc 60
tgtctccgcc agcagaatgt aaactccatg aggtcaggaa tctccgagtt atgttgcgcc 120
agtgtaatcc aagagcccgg aacagtgcct ggcacacagc gggcatatgg aagaacaaat 180
gtgtgaaggt gtgaatgaat gaataattga aagaataaat agtagttctc agcctcacag 240
aacacgggtc acaacctcaa atgacctgct accctgccca taaataacag agatgcagga 300
gtaagtgctg ggctgtgacc tgtcaacatg ctaagccgct caaacaaaac tgcccaacag 360
cccgctggcc gcctatttgc agcactgggc cctgagccgc acattcccat ttcgttgata 420
aagaaactga ccagatagtt taagtggcct gctgcggaag acagagctgg tgctgcaccg 480
gtcgctgctt ccccagtcct tttttggcct cctttctgac gcgacgcaga ccccagttct 540
ggagagtctg tcactcgctc cccgtggtgg gagatcagag gcctggtgtc cttgggagcg 600
gcgagcggtg ctcggcgcag gatagaaagg gagtgcgcgc ccgagtcccc cagatccctg 660
ggaacccgcg ccaccctccc gcccctgccc atccccggcc gcgctgtcag tctccattag 720
cgctaacagg ctccagacgg agcgggccgg gcgctgggtt aatgcaatcg gcgcgttacc 780
tggggcgcag gctacattac cagcccggcc cccgccaggc acggccagaa ccagtcagcc 840
cgcgccctgc cggccgcccc gcgcctccag ctcttccccg gccccgcccg aacgccacac 900
ggcggagccc agccccagcc cgcgccctag agcctgccaa ggcgccgccg gtcgggggcc 960
ggcagggcgc aaggcaccag ggatcccctc gccgccggac acgtgagtgc gccctgagcg 1020
cgggacaggg ctaggtctgc ctgggaggcc cgggccgaga cgcgccagca gagggctagc 1080
gagtttgtag tgcagtgacg ttaagtgtcc gagaaggctc ctgtggctgt tgaagtgtcg 1140
cggacctgag ctggggaggg ggtcggcacg ctgccctcag cctcggtgag ttcaatccca 1200
gccatttggg gcaggcgaga gtgggtgaac gaggaaaagt gctgcagggt cttcagccgc 1260
ccccagaggg ctgtcagaag tctccaactc ttgagttccg gcgtgcccca acctctgttt 1320
ccaaattttt ccagcggacg cgcgctcttt tctgggaacc ctgcgtccgc tcagcgcgcg 1380
ctcatcccag tgtctaaggc gctcccgggt ggtcttggga gttgcaagta gggaggaacg 1440
gccgggtaac cacctctttt ccctttatcc aagcagagcc tcggcgtgcc cccaggaccg 1500
gtaaagttcc tctcgccagc cgcatccatg cttctggcgc ggatgaaccc gcaggtgcag 1560
cccgagaaca acggggcgga cacgggtcca gagcagcccc ttcgggcgcg caaaactgcg 1620
gagctgctgg tggtgaagga gcgcaacggc gtccagtgcc tgctggcgcc ccgcgacggc 1680
gacgcgcagc cccgggagac ctggggcaag aagatcgact tcctgctgtc cgtagtcggc 1740
ttcgcagtgg acctggccaa cgtgtggcgc ttcccctacc tctgctacaa gaacggcggc 1800
ggtgagcgtg gggtcgggct gggaatttga atctgggagg tccactgtct gcagcggtgg 1860
ctgggacagg agctggaata cacacggaag ggaggcgagg agacaggggc aaatctgggg 1920
cgcagaaaga actggacagg gctaacggga aaaaaaaaag attggagtcc tctggaaggt 1980

cattttccca ggctctttgc agagtacctc gagctcattc cagcggaagt gtcaggattg 2040
ggcaccctgg aagcaaaaca gcagaagagt gaaatcgagt catgacccta aagtcatggt 2100
aggggtatgg atggaaagga cagaatctgg ggtgccaggt tgggtggggg agcctgacct 2160
tttgatggtc tgctggaagg gaggtggaga ttccaagagc 2200

<210> 17
<211> 98
<212> DNA
<213> Artificial Sequence

<220>
<223> Designed methylated oligonucleotide for experiment

<220>
<221> modified_base
<222> (20)..(20)
<223> n stands for m5c
<220>
<221> modified_base
<222> (23)..(23)
<223> n stands for m5c
<220>
<221> modified_base
<222> (31)..(31)
<223> n stands for m5c
<220>
<221> modified_base
<222> (46)..(46)
<223> n stands for m5c
<220>
<221> modified_base
<222> (51)..(51)
<223> n stands for m5c
<220>
<221> modified_base
<222> (53)..(53)
<223> n stands for m5c
<220>
<221> modified_base
<222> (55)..(55)
<223> n stands for m5c

<400> 17
gttggccact gcggagtcgn gcngggtggc nggccgcacc tacagngccg ngngngcggt 60
gagcctggcc gtgtggggga tcgtcacact cgtcgtgc 98

<210> 18
<211> 98
<212> DNA
<213> Artificial Sequence

<220>
<223> Designed unmethylated methylated oligonucleotide for experiment

<400> 18
gttggccact gcggagtcgc gccgggtggc cggccgcacc tacagcgccg cgcgcgcggt 60
gagcctggcc gtgtggggga tcgtcacact cgtcgtgc 98

<210> 19
<211> 14
<212> DNA
<213> Artificial Sequence

<220>
<223> Designed oligonucleotide for experiment

<400> 19
gccacccggc gcga                                                          14

<210> 20
<211> 20
<212> DNA
<213> Artificial Sequence

<220>
<223> Designed biotinated oligonucleotide for fixation

<400> 20
gcacgacgag tgtgacgatc                                                    20

<210> 21
<211> 18
<212> DNA
<213> Artificial Sequence

<220>
<223> Designed oligonucleotide primer for PCR

<400> 21
ttggccactg cggagtcg                                                      18

<210> 22
<211> 20
<212> DNA
<213> Artificial Sequence

<220>
<223> Designed oligonucleotide primer for PCR

<400> 22
gcacgacgag tgtgacgatc                                                    20

<210> 23
<211> 98
<212> DNA
<213> Artificial Sequence

<220>
<223> Designed oligonucleotide consisting of objective DNA domain (GPR7-
2079-2176, methylated cytosin is also shown as C)

<400> 23
gttggccact gcggagtcgc gccgggtggc cggccgcacc tacagcgccg cgcgcgcggt    60
gagcctggcc gtgtggggga tcgtcacact cgtcgtgc                            98